# EUROPEAN PATENT SPECIFICATION

(11) **EP 2 198 304 B1**
(45) Date of publication and mention of the grant of the patent: **13.01.2016**
(21) Application number: 08829631.4
(22) Date of filing: 06.09.2008
(51) Int. Cl.: G01N 33/573, A61K 38/46, A61K 39/395, A61K 39/42, C07K 14/00

(54) **MOLECULES AND METHODS FOR TREATMENT AND DETECTION OF DIABETES**
MOLEKÜLE UND VERFAHREN ZUR BEHANDLUNG UND ZUM NACHWEIS VON DIABETES
MOLÉCULES ET PROCÉDÉS DE TRAITEMENT ET DE DÉTECTION DU DIABÈTE

(30) Priority: 06.09.2007 US 970409 P
(43) Date of publication of application: 23.06.2010
(73) Proprietor: University of Washington, Seattle, WA 98105-4608 (US)
(72) Inventor: HAMPE, Christiane, S., Seattle, WA 98115 (US); LERNMARK, Ake, Seattle, WA 98195 (US); OAK, Shilpa, Seattle, WA 98115 (US)
(74) Representative: Cornish, Kristina Victoria Joy
(86) International application number: PCT/US2008/075524
(87) International publication number: WO 2009/033121

(56) References cited:
- SCHLOSSER M ET AL: "Dynamic changes of GAD65 autoantibody epitope specificities in individuals at risk of developing type 1 diabetes", DIABETOLOGIA ; CLINICAL AND EXPERIMENTAL DIABETES AND METABOLISM, SPRINGER, BERLIN, DE, vol. 48, no. 5, 1 May 2005 (2005-05-01), pages 922-930, XP019322528, ISSN: 1432-0428, DOI: 10.1007/S00125-005-1719-1
- TREMBLE J ET AL: "Human B Cells Secreting Immunoglobulin G to Glutamic Acid Decarboxylase-65 from a Nondiabetic Patient with Multiple Autoantibodies and Graves'Disease: A Comparison with Those Present in Type 1 Diabetes", JOURNAL OF CLINICAL ENDOCRINOLOGY AND METABOLISM, THE ENDOCRINE SOCIETY, US, vol. 82, no. 8, 1 January 1997 (1997-01-01), pages 2664-2670, XP008124612, ISSN: 0021-972X, DOI: 10.1210/JC.82.8.2664
- BINDER K A ET AL: "Epitope analysis of GAD65Ab using fusion proteins and rFab", JOURNAL OF IMMUNOLOGICAL METHODS, ELSEVIER SCIENCE PUBLISHERS B.V.,AMSTERDAM, NL, vol. 295, no. 1-2, 1 December 2004 (2004-12-01), pages 101-109, XP004701196, ISSN: 0022-1759, DOI: 10.1016/J.JIM.2004.09.015
- TYLER R. HALL ET AL: "Modulation of diabetes in NOD mice by GAD65-specific monoclonal antibodies is epitope specific and accompanied by anti-idiotypic antibodies", IMMUNOLOGY, vol. 123, no. 4, 1 April 2008 (2008-04-01) , pages 547-554, XP55026817, ISSN: 0019-2805, DOI: 10.1111/j.1365-2567.2007.02724.x
- S. OAK ET AL: "The lack of anti-idiotypic antibodies, not the presence of the corresponding autoantibodies to glutamate decarboxylase, defines type 1 diabetes", PROCEEDINGS OF THE NATIONAL ACADEMY OF SCIENCES, vol. 105, no. 14, 8 April 2008 (2008-04-08), pages 5471-5476, XP55026816, ISSN: 0027-8424, DOI: 10.1073/pnas.0800578105
- MENARD ET AL: "Anti-GAD monoclonal antibody delays the onset of diabetes mellitus in NOD mice.", PHARMACEUTICAL RESEARCH, vol. 16, no. 7, 1 July 1999 (1999-07-01), pages 1059-1066, XP55027086, ISSN: 0724-8741
- VERGE ET AL: "Number of autoantibodies (against insulin, GAD or ICA512/IA2) rather than particular autoantibody specificities determines risk of type I diabetes.", JOURNAL OF AUTOIMMUNITY, vol. 9, no. 3, 1 June 1996 (1996-06-01), pages 379-383, XP55027024, ISSN: 0896-8411
- GORUS ET AL: "IA-2-autoantibodies complement GAD65-autoantibodies in new-onset IDDM patients and help predict impending diabetes in their siblings. The Belgian Diabetes Registry.", DIABETOLOGIA, vol. 40, no. 1, 1 January 1997 (1997-01-01), pages 95-99, XP55027041, ISSN: 0012-186X
- VANDEWALLE C L ET AL: "Associations of GAD65- and IA-2- autoantibodies with genetic risk markers in new-onset IDDM patients and their siblings. The Belgian Diabetes Registry.", DIABETES CARE OCT 1997 LNKD- PUBMED:9314633, vol. 20, no. 10, October 1997 (1997-10), pages 1547-1552, XP002675934, ISSN: 0149-5992
- XIN WANG ET AL: "Anti-Idiotypic Antibody Specific to GAD65 Autoantibody Prevents Type 1 Diabetes in the NOD Mouse", PLOS ONE, vol. 7, no. 2, 1 January 2012 (2012-01-01) , pages E32515-E32515, XP55026812, ISSN: 1932-6203, DOI: 10.1371/journal.pone.0032515
- E. ORTQVIST ET AL: "Changes in GAD65Ab-Specific Antiidiotypic Antibody Levels Correlate with Changes in C-Peptide Levels and Progression to Islet Cell Autoimmunity", JOURNAL OF CLINICAL ENDOCRINOLOGY & METABOLISM, vol. 95, no. 11, 1 November 2010 (2010-11-01), pages E310-E318, XP55026814, ISSN: 0021-972X, DOI: 10.1210/jc.2010-0785
- PADOA. C. ET AL.: 'Recombinant Fabs of Human Monoclonal Antibodies Specific to the Middle Epitope ofGAD65 Inhibit Type 1 Diabetes-Specific GAD65Abs' DIABETES vol. 52, no. 11, November 2003, pages 2689 - 2695, XP008132271
- FENALTI.G. ET AL.: 'Molecular characterization of a disease associated conformational epitope on GAD65 recognised by a human monoclonal antibody b96.11.' MOL IMMUNOL. vol. 44, no. 6, February 2007, pages 1178 - 1189, XP005664734
- GILLIAM. L. ET AL.: 'Multiplicity of the antibody response to GAD65 in Type I diabetes' CLIN EXP IMMUNOL. vol. 138, no. 2, November 2004, pages 337 - 341, XP008132269
- RUI M. ET AL.: 'Species and epitope specificity of two 65 kDa glutamate decarboxylase time- resolved fluorometric immunoassays' JOURNAL OF IMMUNOLOGICAL METHODS vol. 319, January 2007, pages 133 - 143, XP005835615

## Description

### TECHNICAL FIELD OF THE INVENTION

The invention provides compositions for treatment and prevention of Type 1 diabetes.

### BACKGROUND OF THE INVENTION

Type 1 diabetes (T1 D) is an autoimmune disease that is characterized by the presence of serologically detectable autoantibodies to multiple islet cell autoantigens. Autoantibodies to GAD65 (GAD65Ab) can be detected in the majority of new onset T1D patients, in patients with latent autoimmune diabetes in adults (LADA), diabetes-related polyendocrine diseases, and in some rare neurologic diseases notably Stiff Person Syndrome (SPS). GAD65Ab are only rarely detected in healthy individuals (-1%), and since they often herald onset of T1D by months or years, they are used to predict disease together with other islet autoantibodies.

The function of these autoantibodies in the pathogenesis of T1D is poorly defined. While GAD65Ab are mainly recognized as an epiphenomenon resulting from the autoimmune destruction of the pancreatic beta cells, some studies suggest that the autoantibodies may be involved in antigen processing and presentation and thus modulate the immune response.

GAD65Ab persist for years after the onset of diabetes, with a remarkably stable epitope pattern. This is intriguing because the beta cells as the antigen source decline rapidly after onset of disease. It has been proposed that continuous beta cell regeneration or protein mimicry may explain this phenomenon of Ab persistence. It is also possible that an antigen- independent memory could be generated by the interaction of GAD65Ab-specific B cells with complementary B cells that produce anti-idiotypic antibodies (anti-Id). Tolerization against the beta cell autoantigens GAD65 and insulin has been attempted as a method for the prevention of the disease. GAD65 administration prevents beta cell destruction and the resulting diabetes in the NOD mouse, stressing the importance of GAD65 as an autoantigen in the disease progression of the NOD mouse; even though GAD65 is present only in very limited amounts in murine beta cells. Recently, alum-formulated GAD65 has been used in phase II clinical trials in GAD65 antibody positive diabetes patients. While there were no adverse events immunomodulation with GAD65 resulted in a reduced loss of endogenous insulin production. GAD65 administration in preclinical NOD mouse and clinical phase II trials has been suggested to result in tolerization of the T cell-mediated immune response. However, these results need confirmation in larger clinical trials.

While autoantibodies to GAD65 (GAD65Ab) can be detected in the majority of new onset T1D patients, the presence of GAD65Ab in the NOD mouse remains controversial. Moreover, as in human T1D, the role of GAD65Ab in the pathogenesis is poorly defined. While it is established that these antibodies are valuable markers for the disease progression, they are mainly viewed as innocent bystanders, resulting from the destruction of the pancreatic beta cells. However, recent studies suggest that GAD65Ab are involved in GAD65 processing and presentation and thus may modulate the immune response. The effect of GAD65Ab on the disease progression in NOD mice was first studied by administering GAD65-specific monoclonal antibody GAD-6. The observed delayed onset of T1D in response to this treatment was attributed to possible impaired recognition of GAD65 by antigen-specific T cells, (Menard et al, Pharmaceutical Research 1999, Vol. 16, no.7. pages 1059-1066).

There remains a need for methods of identifying subjects at greatest risk for developing T1D, and also for treating T1D. The invention described herein addresses these needs and others by providing compositions for effective treatment of T1D.

### SUMMARY OF THE INVENTION

The subject matter for which protection is sought is as defined in the claims. In one aspect, the present invention provides a pharmaceutical composition comprising an anti-idiotypic antibody that specifically recognizes and binds an epitope of antibody b96.11 and, optionally, a pharmaceutically acceptable carrier, wherein the anti-idiotypic antibody competitively inhibits binding of b96.11 to GAD65 , wherein said antibody b96.11 has a light chain comprising the amino acid sequence shown in SEQ ID NO: 1 and a heavy chain comprising the amino acid sequence shown in SEQ ID NO: 3.

In one embodiment the invention provides a pharmaceutical composition comprising a nucleic acid molecule that encodes the anti-idiotypic antibody and, optionally, a pharmaceutically acceptable carrier.

The composition of the invention may be used in delaying the onset of diabetes, inhibiting insulitis or inducing an anti-idiotypic immune response in a subject. Optionally the subject is a first degree relative of a Type 1 diabetes patient.

The disclosure provides molecules that specifically recognize and bind the epitope of glutamate decarboxylase (GAD65) that is bound by antibody b96.11. This epitope is located within amino acid residues 308-365 of GAD65. Such molecules are referred to herein as antibodies. The invention additionally provides molecules that specifically recognize and bind antibody b96.11. These molecules are capable of competing with GAD65 for binding with b96.11, and competitively inhibit such binding. These latter molecules are referred to herein as anti-idiotypic antibodies (anti-Id).

These antibodies can be provided in the form of a pharmaceutical composition comprising the antibody and, optionally, a pharmaceutically acceptable carrier. Such antibodies can be monoclonal or polyclonal and include chimeric antibodies, recombinant molecules, fusion proteins and antibody fragments, so long as they retain the ability to specifically recognize and bind the epitope of GAD65 that is bound by antibody b96.11, or in the case of anti- idiotypic antibodies, so long as they retain the ability to compete with GAD65 for binding to b96.11. Also contemplated by the invention are nucleic acid molecules that encode an anti-idiotypic antibody of the invention.

The antibodies and anti-idiotypic antibodies described herein can be used in methods for delaying the onset of Type 1 diabetes and for inhibiting insulitis and other diabetic symptoms. The methods comprise administering to a subject a composition of the invention. The composition comprises an anti-idiotypic antibody of the invention or a nucleic acid molecule encoding an anti-idiotypic antibody of the invention.

The description further provides methods for detecting a susceptibility to Type 1 diabetes in a subject and for detecting the presence of anti-idiotypic antibodies to GAD65. The method comprises contacting a specimen with a molecule (such as an antibody) that specifically recognizes and binds the epitope of glutamate decarboxylase (GAD65) that is bound by antibody b96.11. The method further comprises detecting binding of the molecule to the specimen. The absence (or relative absence) of binding is indicative of susceptibility to Type 1 diabetes and of the absence of anti-idiotypic antibodies. In a typical embodiment, the molecule is an antibody. The antibody or other molecule can, optionally, be labeled with a detectable marker.

### BRIEF DESCRIPTION OF THE FIGURES

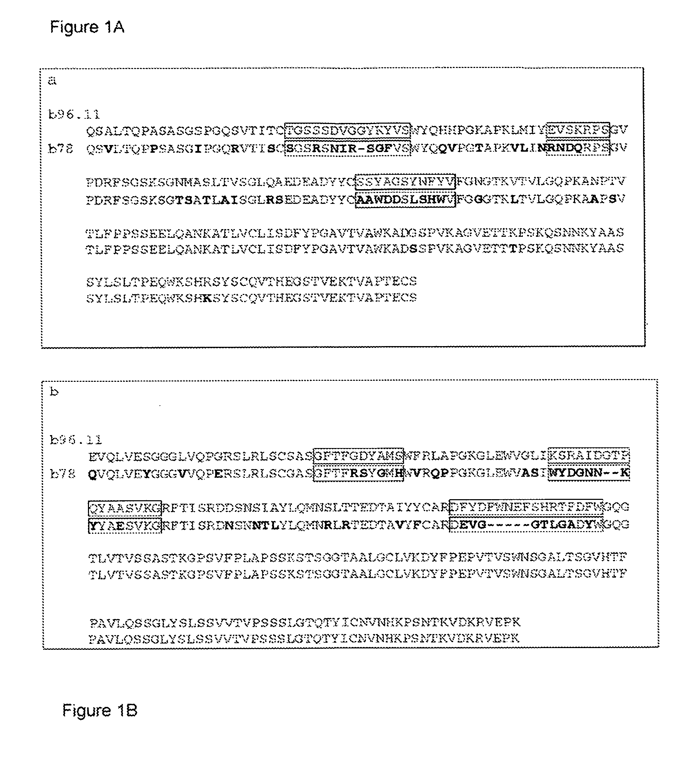

Figures 1A-1B: Alignment of light (1A; SEQ ID NO:1,2) and heavy (B; SEQ ID NO: 3,4) chains of monoclonal antibodies b96.11 and b78 (accession numbers 917308 and 917304, respectively). Complementarity determining regions (CDR) used for homology comparison are boxed. Amino acid substitutions between the two antibodies are identified in bold font.

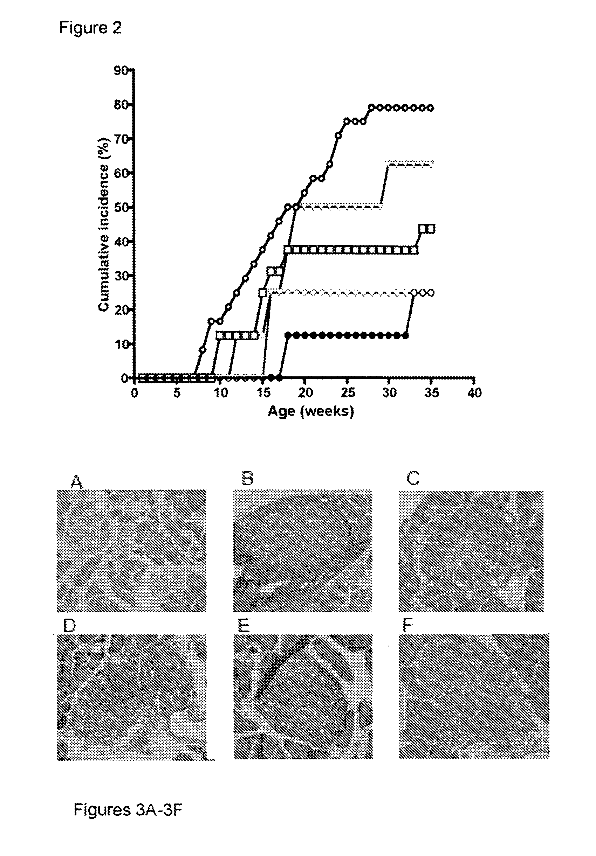

Figure 2: Line graph showing cumulative incidence rate of diabetes development. Control animals were injected with PBS or human polyclonal antibody (white circles). Animals injected with 100 and 50 µg b78 IgG showed no difference in disease progression and were grouped together (white squares). Animals injected with 10, 50, and 100 µg of b96.11 are presented as black inverted triangles, diamonds and circles, respectively. Animals were monitored weekly for diabetes development.
Figures 3A-3F: Photomicrographs showing the histopathology of pancreatic islets at onset of diabetes or at 35 weeks of age. Representative images of pancreatic islets from animals injected with 100 µg (A), 50 µg (B), and 10 µg (C) b96.11 IgG, 100 µg b78 IgG (D), 100 µg human polyclonal IgG (E), and PBS (F). Pancreatic tissues were sectioned and stained with hematoxylin and eosin.

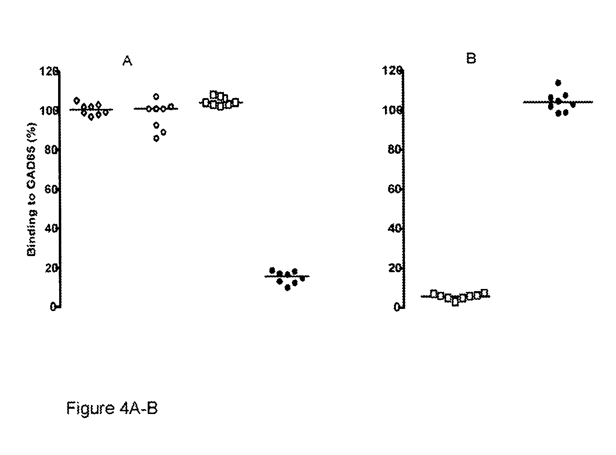

Figures 4A-4B: Effect of mouse sera on GAD65Ab reactivity with GAD65. B96.11 (4A) and b78 (4B) were incubated at their half-maximal binding concentration with sera of animals injected with 100 µg b96.11 (black circles), b78 (white squares), human polyclonal IgG (white circles), or PBS (white diamonds). The binding capacity was determined in a competitive RBA and is presented as percent binding (non-competed binding equals 100%).

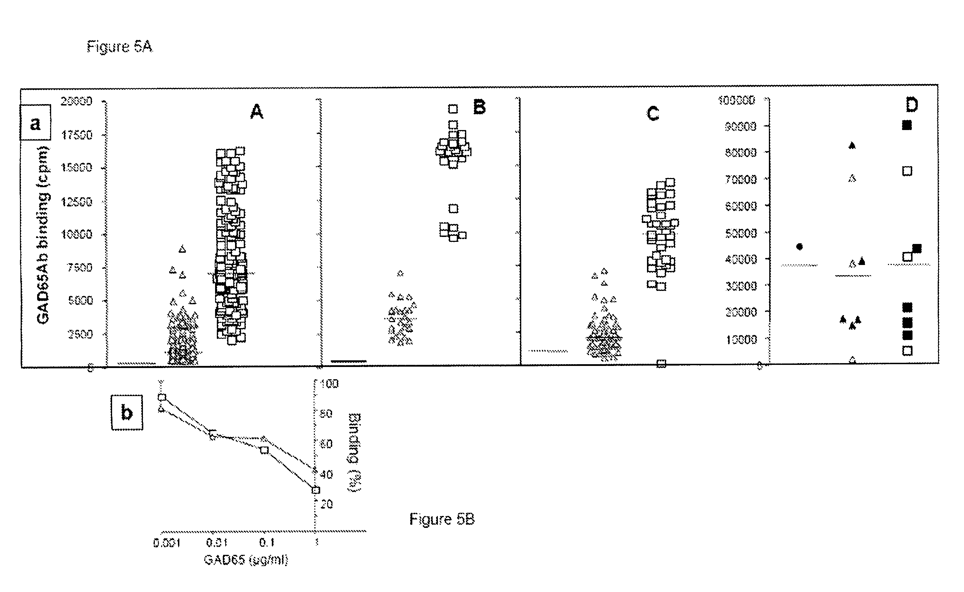

Figure 5A: Scatterplots showing that sera of healthy individuals and FDR contain GAD65Ab/inhibitor complexes, while T1D patients and SPS patients lack inhibitors to disease-specific GAD65Ab. Sera of healthy individuals (panel A) and First Degree Relatives (FDR) (panel B) that tested negative for GAD65Ab in conventional RIA, and sera from GAD65Ab-positive T1D patients (C) and SPS patients (D) were absorbed on immobilized b96.11-PAS and b78-PAS. The serum samples and the flow-through of beads were tested for the presence of GAD65Ab in a RIA. Binding to GAD65 before (circles) and after absorption to b96.11 (triangles) or b78 (squares) is shown in cpm. SPS patients that are also diagnosed with T1D are represented by the black symbols. Median binding is indicated.
Figure 5B: Graph showing results of an ELISA of isolated inhibitor from a healthy individual. Binding of inhibitor isolated from b96.11-PAS or b78-PAS after absorption with serum from a healthy individual to b96.11-HRP (triangles) and b78-HRP (squares) was analyzed in the presence of the indicated concentrations of human recombinant GAD65. Binding is shown as percent binding with binding in the absence of GAD65 set as 100%

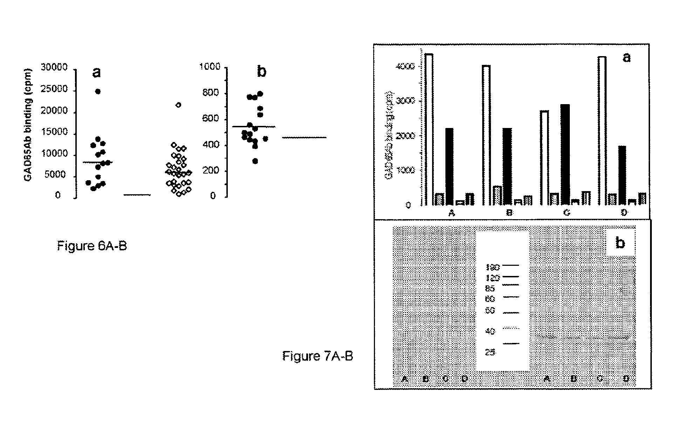

Figure 6A: Scatterplot showing that the inhibitor is specific to GAD65Ab. GAD65 binding by unmasked sera is competed by recombinant human GAD65 and not by BSA. Sera of FDR absorbed by b36.11-PAS were tested for binding to ^{[35]}S-GAD65 in the presence of recombinant human GAD65 (150 mM) (open circles), or BSA (150 nM) (open diamonds). Median binding is indicated.
Figure 6B: Scatterplot showing that absorption of inhibitors that reduce GAD65Ab binding is specific to GAD65Ab and cannot be achieved by an irrelevant human monoclonal antibody. Sera of healthy individuals (n=15) and FDR (n=15) were absorbed to immobilized HAA1. The flow-through was tested for binding to GAD65. GAD65Ab titer before (open circles) and after (black circles) absorption is shown in cpm. Median binding is indicated.
Figure 7A: Bar graph showing that GAD65Ab and inhibitors are present in the sera's Ig-fraction only. Purified Ig and depleted serum were prepared from four FDR (A, B, C, D). Absorbed sera (white), purified Ig before (grey) and after absorption (black), Ig-depleted sera before (horizontal) and after absorption (vertical) were tested for binding to GAD65. The data show that affinity purification of GAD65Ab-negative sera on b96.11-PAS leads to the dissociation of the immune complexes to reveal the presence of hitherto undetectable GAD65Ab.
Figure 7B: Western blot from analysis of serum fractions described in Figure 7A. The *Ig-*depleted serum fractions (left of the molecular weight markers) and purified Ig fractions (right of the molecular weight markers) were tested with goat-anti human kappa chain. Molecular weight markers are shown.

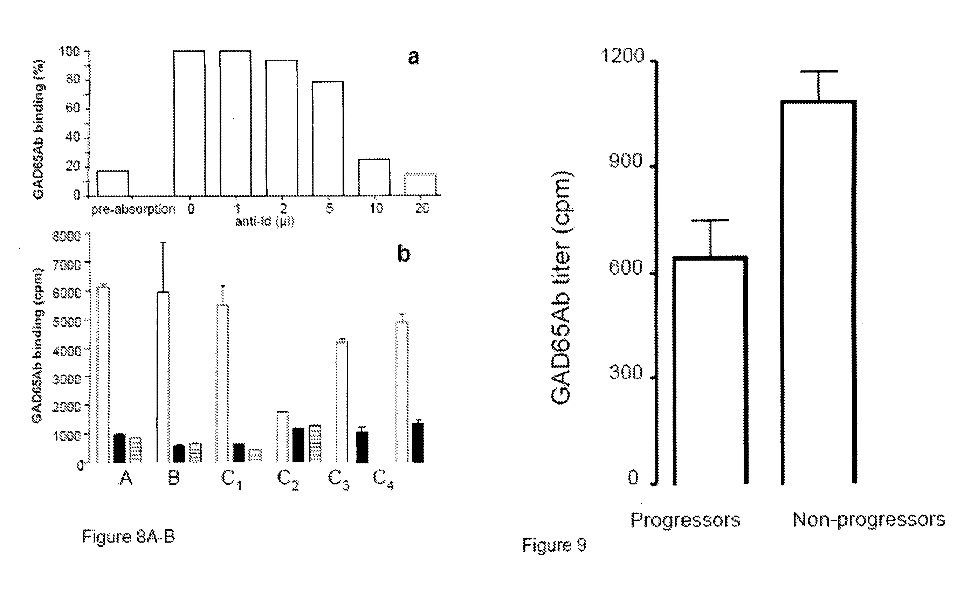

Figure 8A: Bar graph showing percent GAD65Ab binding. Anti-Id are cross-inhibitory between healthy individuals and FDR and inhibit GAD65 binding by T1D patients' sera. Anti-Id was isolated from a FDR as described and added to the un-masked serum at the indicated concentrations. GAD65 binding is shown as percent binding (binding of absorbed, uninhibited serum is set to 100%). A dose-dependent inhibition of GAD65 binding was observed.
Figure 8B: Bar graph showing GAD65Ab binding reported in cpm. Anti-Id was isolated from a healthy individual as described. Anti-Id (20 µl) from the healthy individual (black) and the FDR (horizontal) were added to unmasked sera of a healthy individual (A), FDR (B), and unabsorbed sera of four T1D patients (C₁₋₄).
Figure 9: Bar graph showing that individuals that later progress to T1D have less b96.11-specific anti-Id as compared to non-progressors. Sera of GAD65Ab-negative non-progressors (n=33) and progressors (n=6) were absorbed to immobilized b96. 11-PAS. The GAD65Ab of the run-through was then tested.

### DETAILED DESCRIPTION OF THE INVENTION

The invention is based on the surprising discovery that the lack of anti-idiotypic antibodies to the b96.11 monoclonal antibody specificity, rather than GAD65Ab per se, is a characteristic of Type 1 diabetes (T1D). T1D is an autoimmune disease characterized by the presence of serologically detectable autoantibodies to multiple islet cell autoantigens. Autoantibodies to GAD65 (GAD65Ab) can be detected in the majority of new onset T1D patients, but rarely in the general population. GAD65Ab often herald the onset of T1D by months or years and are used to predict disease together with other islet cell autoantibodies. The studies described herein show that: (1) healthy individuals and first-degree relatives (FDR) of T1D patients that tested GAD65Ab-negative in conventional screening assays presented GAD65Ab after preabsorption with GAD65-reactive monoclonal antibodies (mAbs), suggesting that these GAD65Ab are present, but masked by epitope-specific anti-ld; (2) GAD65Ab-positive T1D patients show a specific lack of anti-Id to disease-associated GAD65Ab, b96.11, possibly releasing these epitope GAD65Ab specificities to the circulation; and (3) GAD65Ab-negative individuals that later progressed to T1D show significantly lower levels of b96.11-specific anti-Id as compared to the non-progressors. Accordingly, it is the absence of these anti-Id, rather than the presence of GAD65Ab, that is characteristic for T1D. Individuals that progress to T1D may have an early lack of anti-Id suggesting a predictive value. Screening of sera for anti-Id specific to disease-associated GAD65Ab provides a valuable tool in the characterization and risk assessment for T1 D. The invention provides materials that can be used to modulate diabetes.

### Definitions

All scientific and technical terms used in this application have meanings commonly used in the art unless otherwise specified. As used in this application, the following words or phrases have the meanings specified.

As used herein, "absence" of, for example, anti-idiotypic antibodies, means significantly lower levels in a test sample as compared to normal samples.

As used herein, "polypeptide" includes proteins, fragments of proteins, and peptides, whether isolated from natural sources, produced by recombinant techniques or chemically synthesized. Peptides of the invention typically comprise at least about 6 amino acids.

As used herein, "vector" means a construct, which is capable of delivering, and preferably expressing, one or more gene(s) or sequence(s) of interest in a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells.

As used herein, "expression control sequence" means a nucleic acid sequence that directs transcription of a nucleic acid. An expression control sequence can be a promoter, such as a constitutive or an inducible promoter, or an enhancer. The expression control sequence is operably linked to the nucleic acid sequence to be transcribed.

The term "nucleic acid" or "polynucleotide" refers to a deoxyribonucleotide or ribonucleotide polymer in either single- or double-stranded form, and unless otherwise limited, encompasses known analogs of natural nucleotides that hybridize to nucleic acids in a manner similar to naturally-occurring nucleotides.

An "immunogenic peptide," as used herein is a portion of a protein that is recognized (i.e., specifically bound) by a B-cell and/or T-cell surface antigen receptor. Such immunogenic peptides generally comprise at least 5 amino acid residues, more preferably at least 10 amino acid residues of a protein associated with cancer. Typically, an immunogenic peptide comprises 15 to 18 amino acid residues. In some embodiments, immunogenic peptides include peptides in which an N-terminal leader sequence and/or transmembrane domain have been deleted. Other immunogenic peptides may contain a small N- and/or C-terminal deletion (e.g., 1-30 amino acids, preferably 5-15 amino acids), relative to the mature protein.

As used herein, "pharmaceutically acceptable carrier" includes any material which, when combined with an active ingredient, allows the ingredient to retain biological activity and is non-reactive with the subject's immune system. Examples include, but are not limited to, any of the standard pharmaceutical carriers such as a phosphate buffered saline solution, water, emulsions such as oil/water emulsion, and various types of wetting agents. Preferred diluents for aerosol or parenteral administration are phosphate buffered saline or normal (0.9%) saline.

Compositions comprising such carriers are formulated by well known conventional methods (see, for example, Remington's Pharmaceutical Sciences, 18th edition, A. Gennaro, ed., Mack Publishing Co., Easton, PA, 1990).

As used herein, "adjuvant" includes those adjuvants commonly used in the art to facilitate an immune response.

As used herein, "a" or "an" means at least one, unless clearly indicated otherwise.

### Polynucleotides of the Invention

The invention provides polynucleotides that encode one or more antibodies of the invention or portions thereof. Preferred polynucleotides comprise at least 15 consecutive nucleotides, preferably at least 30 consecutive nucleotides. Polynucleotides that are fully complementary to any such sequences are also encompassed by the present invention. Polynucleotides may be single-stranded (coding or antisense) or double-stranded, and may be DNA (genomic, cDNA or synthetic) or RNA molecules. RNA molecules include siRNA (discussed below), HnRNA molecules, which contain introns and correspond to a DNA molecule in a one-to-one manner, and mRNA molecules, which do not contain introns. Additional coding or non-coding sequences may, but need not, be present within a polynucleotide of the present invention and a polynucleotide may, but need not, be linked to other molecules and/or support materials. Portions of polynucleotides can be useful as primers and probes for the amplification and detection of related molecules in tissue specimens.

Polynucleotides may comprise a native sequence or may comprise a variant of such a sequence. Polynucleotide variants contain one or more substitutions, additions, deletions and/or insertions such that the immunogenicity of the encoded polypeptide is not diminished, relative to a native protein. Variants preferably exhibit at least about 70% identity, more preferably at least about 80% identity and most preferably at least about 90% identity to a polynucleotide sequence that encodes a native protein or a portion thereof.

Two polynucleotide or polypeptide sequences are said to be "identical" if the sequence of nucleotides or amino acids in the two sequences is the same when aligned for maximum correspondence as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, or 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc. Madison, WI), using default parameters. This program embodies several alignment schemes described in the following references: Dayhoff, M.O. (1978) A model of evolutionary change in proteins-Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) CABIOS 5:151-153; Myers, E.W. and Muller W. (1988) CABIOS 4:11-17; Robinson, E.D. (1971) Comb. Theor. 11:105; Santou, N., Nes, M. (1987) Mol. Biol, Evol. 4:406-425; Sneath, P.H.A. and Sokal, R.R. (1973) Numerical Taxonomy the Principles and Practice of Numerical Taxonomy, Freeman Press, San Francisco, CA; Wilbur, W.J. and Lipman, D.J. (1983) Proc. Natl. Acad. Sci. USA 80:726-730.

Preferably, the "percentage of sequence identity" is determined by comparing two optimally aligned sequences over a window of comparison of at least 20 positions, wherein the portion of the polynucleotide or polypeptide sequence in the comparison window may comprise additions or deletions (i.e. gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (i.e. the window size) and multiplying the results by 100 to yield the percentage of sequence identity.

Variants may also, or alternatively, be substantially homologous to a native gene, or a portion or complement thereof. Such polynucleotide variants are capable of hybridizing under moderately stringent conditions to a naturally occurring DNA sequence encoding a native IGF-related protein (or a complementary sequence).

Suitable "moderately stringent conditions" include prewashing in a solution of 5 X SSC, 0.5% SDS 1.0 mM EDTA (pH 8.0); hybridizing at 50°C-55°C, 5 X SSC, overnight: followed by washing twice at 65°C for 20 minutes with each of 2X, 0.5X and 0.2X SSC containing 0. 1 % SDS.

As used herein, "highly stringent conditions" or "high stringency conditions" are those that (1) employ low ionic strength and high temperature for washing, for example 0.015 M sodium chlorine/0.0015 M sodium citrate/0.1% sodium dodecyl sulfate at 50°C: (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C. The skilled artisan will recognize how to adjust the temperature, ionic strength, etc. as necessary to accommodate factors such as probe length and the like.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

Polynucleotides may be prepared using any of a variety of techniques known in the art. DNA encoding an antibody or portion thereof may be obtained from a cDNA library prepared from tissue expressing a corresponding protein mRNA. Libraries can be screened with probes (such as antibodies or oligonucleotides of at least about 20-80 bases) designed to identify the gene of interest or the protein encoded by it. Screening the cDNA or genomic library with the selected probe may be conducted using standard procedures, such as those described in Sambrook et al., Molecular Cloning: A Laboratory Manual (New York: Cold Spring Harbor Laboratory Press, 1989). An alternative means to isolate the gene encoding an IGF-related protein is to use PCR methodology (Sambrook et al., *supra;* Dieffenbach et al., PCR Primer: A Laboratory Manual (Cold Spring Harbor Laboratory Press, 1995)).

The oligonucleotide sequences selected as probes should be sufficiently long and sufficiently unambiguous that false positives are minimized. The oligonucleotide is preferably labeled such that it can be detected upon hybridization to DNA in the library being screened. Methods of labeling are well known in the art, and include the use of radiolabels, such as ³²P-labeled ATP, biotinylation or enzyme labeling. Hybridization conditions, including moderate stringency and high stringency, are provided in Sambrook et al., *supra.*

Polynucleotide variants may generally be prepared by any method known in the art, including chemical synthesis by, for example, solid phase phosphoramidite chemical synthesis. Modifications in a polynucleotide sequence may also be introduced using standard mutagenesis techniques, such as oligonucleotide-directed site-specific mutagenesis (see Adelman et al., DNA 2:183, 1983). Alternatively, RNA molecules may be generated by *in vitro* or *in vivo* transcription of DNA sequences encoding an IGF-related protein, or portion thereof, provided that the DNA is incorporated into a vector with a suitable RNA polymerase promoter (such as T7 or SP6). Certain portions may be used to prepare an encoded polypeptide, as described herein. In addition, or alternatively, a portion may be administered to a patient such that the encoded polypeptide is generated *in vivo* (e.g., by transfecting antigen-presenting cells, such as dendritic cells, with a cDNA construct encoding an IGF-related polypeptide, and administering the transfected cells to the patient).

Any polynucleotide may be further modified to increase stability *in vivo.* Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine.

Nucleotide sequences can be joined to a variety of other nucleotide sequences using established recombinant DNA techniques. For example, a polynucleotide may be cloned into any of a variety of cloning vectors, including plasmids, phagemids, lambda phage derivatives and cosmids. Vectors of particular interest include expression vectors, replication vectors, probe generation vectors and sequencing vectors. In general, a vector will contain an origin of replication functional in at least one organism, convenient restriction endonuclease sites and one or more selectable markers. Other elements will depend upon the desired use, and will be apparent to those of ordinary skill in the art.

Within certain embodiments, polynucleotides may be formulated so as to permit entry into a cell of a mammal, and to permit expression therein. Such formulations are particularly useful for therapeutic purposes, as described below. Those of ordinary skill in the art will appreciate that there are many ways to achieve expression of a polynucleotide in a target cell, and any suitable method may be employed. For example, a polynucleotide may be incorporated into a viral vector such as, but not limited to, adenovirus, adeno-associated virus, retrovirus, or vaccinia or other pox virus (e.g., avian pox virus). Techniques for incorporating DNA into such vectors are well known to those of ordinary skill in the art. A retroviral vector may additionally transfer or incorporate a gene for a selectable marker (to aid in the identification or selection of transduced cells) and/or a targeting moiety, such as a gene that encodes a ligand for a receptor on a specific target cell, to render the vector target specific. Targeting may also be accomplished using an antibody, by methods known to those of ordinary skill in the art.

Other formulations for therapeutic purposes include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle *in vitro* and *in vivo* is a liposome (i.e., an artificial membrane vesicle). The preparation and use of such systems is well known in the art.

### Proteins and Peptides

Proteins and peptides as described herein may be of any length suitable for the intended use. For example, additional sequences derived from the native protein and/or heterologous sequences may be present, and such sequences may, but need not, possess further ligand binding, immunogenic or antigenic properties.

Preferred peptides comprise the sequences of amino acid residues set forth in the tables and figures herein. Adjacent native sequence is not necessary, but small portions (less than 15 additional amino acid residues, preferably less than 10 additional amino acid residues) of adjacent sequence can be used without interfering with the immunogenicity of the peptide or its ease of delivery. Those skilled in the art will appreciate that other portions or variants thereof will be useful. Fragments consisting of about 10, 12, 15, 20, 25, 30, 40, 50, 60, 75 or up to 100 contiguous amino acids of the original sequence are contemplated by the invention. In a typical embodiment, a fragment of the invention comprises an antigen binding region.

Immunogenic peptides may generally be identified using well known techniques, such as those summarized in Paul, Fundamental Immunology, 4th ed., 663-665 (Lippincott-Raven Publishers, 1999) and references cited therein. Such techniques include screening peptides for the ability to react with antigen-specific antibodies, antisera and/or T-cell lines or clones. As used herein, antisera and antibodies are antigen-specific if they specifically bind to an antigen (i.e., they react with the protein in an ELISA or other immunoassay, and do not react detectably with unrelated proteins). Such antisera and antibodies may be prepared using well known techniques. An immunogenic peptide can be a portion of a native protein that reacts with such antisera and/or T-cells at a level that is not substantially less than the reactivity of the full length polypeptide (e.g., in a radioimmunoprecipitation assay, an ELISA and/or T-cell reactivity assay). Such immunogenic portions may react within such assays at a level that is similar to or greater than the reactivity of the full length polypeptide. Such screens may generally be performed using methods well known to those of ordinary skill in the art, such as those described in Harlow and Lane, Antibodies: A Laboratory Manual, Cold Spring Harbor Laboratory, 1988. For example, a peptide may be immobilized on a solid support and contacted with patient sera to allow binding of antibodies within the sera to the immobilized peptide. Unbound sera may then be removed and bound antibodies detected using, for example, ¹²⁵I-labeled Protein A.

A polypeptide "variant", as used herein, is a polypeptide that differs from a native protein in one or more substitutions, deletions, additions and/or insertions, such that the immunogenicity of the polypeptide is not substantially diminished. In other words, the ability of a variant to react with antigen-specific antisera may be enhanced or unchanged, relative to the native protein, or may be diminished by less than 50%, and preferably less than 20%, relative to the native protein. Such variants may generally be identified by modifying one of the above polypeptide sequences and evaluating the reactivity of the modified polypeptide with antigen-specific antibodies or antisera as described herein. Preferred variants include those in which one or more portions, such as an N-terminal leader sequence, have been removed. Other preferred variants include variants in which a small portion (e.g., 1-30 amino acids, preferably 5-15 amino acids) has been removed from the N- and/or C-terminal of the mature protein. Polypeptide variants preferably exhibit at least about 70%, more preferably at least about 90% and most preferably at least about 95% identity (determined as described above) to the identified polypeptides.

Preferably, a variant contains conservative substitutions. A "conservative substitutions" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine: and amino acids with uncharged polar head groups having similar hydrophilicity values include leucine, isoleucine and valine: glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

Polypeptides may comprise a signal (or leader) sequence at the N-terminal end of the protein that co-translationally or post-translationally directs transfer of the protein. The polypeptide may also be conjugated to a linker or other sequence for ease of synthesis, purification or identification of the polypeptide (e.g., poly-FEs), or to enhance binding of the polypeptide to a solid support. For example, a polypeptide may be conjugated to an immunoglobulin Fc region.

Recombinant peptides encoded by DNA sequences as described above may be readily prepared from the DNA sequences using any of a variety of expression vectors known to those of ordinary skill in the art. Expression may be achieved in any appropriate host cell that has been transformed or transfected with an expression vector containing a DNA molecule that encodes a recombinant polypeptide. Suitable host cells include prokaryotes, yeast and higher eukaryotic cells. Preferably, the host cells employed are *E. coli,* yeast, insect cells or a mammalian cell line such as COS or CHO. Supernatants from suitable host/vector systems that secrete recombinant protein or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant polypeptide.

Portions and other variants having fewer than about 100 amino acids, and generally fewer than about 50 amino acids, may also be generated by synthetic means, using techniques well known to those of ordinary skill in the art. For example, such peptides may be synthesized using any of the commercially available solid-phase techniques, such as the Merrifield solid-phase synthesis method, where amino acids are sequentially added to a growing amino acid chain. See Merrifield, J. Am. Chem. Soc. 85:2149-2146, 1963. Equipment for automated synthesis of peptides is commercially available from suppliers such as Perkin Elmer/Applied BioSystems Division (Foster City, CA), and may be operated according to the manufacturer's instructions.

Peptides can be synthesized on a Perkin Elmer/Applied Biosystems Division 430A peptide synthesizer using FMOC chemistry with HPTU (O-BenzotriazoleN,N,N',N'-tetramethyluronium hexafluorophosphate) activation. A Gly-Cys-Gly sequence may be attached to the amino terminus of the peptide to provide a method of conjugation, binding to an immobilized surface, or labeling of the peptide. Cleavage of the peptides from the solid support may be carried out using the following cleavage mixture: trifluoroacetic acid:ethanedithiol:thioanisole:water:phenol (40:1:2:2:3). After cleaving for 2 hours, the peptides may be precipitated in cold methyl-t-butyl-ether. The peptide pellets may then be dissolved in water containing 0.1% trifluoroacetic acid (TFA) and lyophilized prior to purification by C18 reverse phase HPLC. A gradient of 0%-60% acetonitrile (containing 0.1% TFA) in water may be used to elute the peptides. Following lyophilization of the pure fractions, the peptides may be characterized using electrospray or other types of mass spectrometry and by amino acid analysis.

### Fusion Proteins

In some embodiments, the polypeptide is a fusion protein that comprises multiple peptides as described herein, or that comprises at least one peptide as described herein and an unrelated sequence.

Additional fusion partners can be added. A fusion partner may, for example, serve as an immunological fusion partner by assisting in the provision of T helper epitopes, preferably T helper epitopes recognized by humans. As another example, a fusion partner may serve as an expression enhancer, assisting in expressing the protein at higher yields than the native recombinant protein. Certain preferred fusion partners are both immunological and expression enhancing fusion partners. Other fusion partners may be selected so as to increase the solubility of the protein or to enable the protein to be targeted to desired intracellular compartments. Still further fusion partners include affinity tags, which facilitate purification of the protein.

Fusion proteins may generally be prepared using standard techniques, including chemical conjugation. Preferably, a fusion protein is expressed as a recombinant protein, allowing the production of increased levels, relative to a non-fused protein, in an expression system. Briefly, DNA sequences encoding the peptide components may be assembled separately, and ligated into an appropriate expression vector. The 3' end of the DNA sequence encoding one peptide component is ligated, with or without a peptide linker, to the 5' end of a DNA sequence encoding the second peptide component so that the reading frames of the sequences are in phase. This permits translation into a single fusion protein that retains the biological activity of both component peptides.

A peptide linker sequence may be employed to separate the first and the second peptide components by a distance sufficient to ensure that each peptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second peptides; and (3) the lack of hydrophobic or charged residues that might react with the peptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Patent No. 4,935,233 and U.S. Patent No. 4,751,180. The linker sequence may generally be from 1 to about 50 amino acids in length. Linker sequences are not required when the first and second peptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located 5' to the DNA sequence encoding the first peptides. Similarly, stop codons required to end translation and transcription termination signals are present 3<1> to the DNA sequence encoding the second peptide.

Fusion proteins are also provided that comprise a peptide of the present invention together with an unrelated immunogenic protein. Preferably the immunogenic protein is capable of eliciting a memory response. Examples of such proteins include tetanus, tuberculosis and hepatitis proteins (see, for example, Stoute et al., New Engl. J. Med. 336:86-91, 1997).

In general, polypeptides (including fusion proteins) and polynucleotides as described herein are isolated. An "isolated" polypeptide or polynucleotide is one that is removed from its original environment. For example, a naturally occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

### Antibodies

The term "antibody" in the following section includes single anti- GAD65 or anti-b96.11 monoclonal antibodies (including anti-idiotypic antibodies) and anti- GAD65 or b96.11 antibody compositions with polyepitopic specificity. The term "monoclonal antibody" (mAb) as used herein refers to an antibody obtained from a population of substantially homogeneous antibodies, i.e. the antibodies comprising the individual population are identical except for possible naturally-occurring mutations that may be present in minor amounts.

The invention provides antibodies that bind b96.11 proteins and polypeptides. The most preferred antibodies will specifically bind to a b96.11 protein and will not bind (or will bind weakly) to non-b96.11 proteins and polypeptides. Anti-b96.11 antibodies that are particularly contemplated include monoclonal and polyclonal antibodies as well as fragments containing the antigen binding domain and/or one or more complementarity determining regions of these antibodies. As used herein, an antibody fragment is defined as at least a portion of the variable region of the immunoglobulin molecule that binds to its target, i.e., the antigen binding region.

Various methods for the preparation of antibodies are well known in the art. For example, antibodies may be prepared by immunizing a suitable mammalian host using a protein, peptide, or fragment of the invention, in isolated or immunoconjugated form (Antibodies: A Laboratory Manual, CSH Press, Eds., Harlow, and Lane (1988); Harlow, Antibodies, Cold Spring Harbor Press, NY (1989)). In addition, fusion proteins may also be used. In another embodiment, a peptide may be synthesized and used as an immunogen.

The antibodies or fragments may also be produced, using current technology, by recombinant means. Regions that bind specifically to the desired regions of the target protein can also be produced in the context of chimeric or CDR grafted antibodies of multiple species origin. Humanized or human antibodies may also be produced and are preferred for use in therapeutic contexts. Methods for humanizing murine and other non-human antibodies by substituting one or more of the non-human antibody CDRs for corresponding human antibody sequences are well known (see for example, Jones et al., 1986, Nature 321: 522-525; Riechmann et al., 1988, Nature 332: 323-327; Verhoeyen et al., 1988, Science 239: 1534-1536). See also, Carter et al., 1993, Proc. Natl. Acad. Sci. USA 89: 4285 and Sims et al., 1993, J. Immunol. 151: 2296. Methods for producing fully human monoclonal antibodies include phage display and transgenic methods (for review, see Vaughan et al., 1998, Nature Biotechnology 16: 535-539).

Fully human monoclonal antibodies may be generated using cloning technologies employing large human Ig gene combinatorial libraries (i.e., phage display) (Griffiths and Hoogenboom, Building an in vitro immune system: human antibodies from phage display libraries. In: Protein Engineering of Antibody Molecules for Prophylactic and Therapeutic Applications in Man. Clark, M. (Ed.), Nottingham Academic, pp 45-64 (1993); Burton and Barbas, Human Antibodies from combinatorial libraries. Id., pp 65-82). Fully human monoclonal antibodies may also be produced using transgenic mice engineered to contain human immunoglobulin gene loci as described in PCT Patent Application WO98/24893, Kucherlapati and Jakobovits et al., published December 3, 1997 (see also, Jakobovits, 1998, Exp. Opin. Invest. Drugs 7(4): 607-614). This method avoids the in vitro manipulation required with phage display technology and efficiently produces high affinity authentic human antibodies.

Reactivity of antibodies with a protein may be established by a number of well known means, including radioimmunoassay (RIA), western blot, immunoprecipitation, ELISA, and FACS analyses.

As described herein an antibody or fragment thereof may be labeled with a detectable marker or conjugated to a second molecule. Suitable detectable markers include, but are not limited to, a radioisotope, a fluorescent compound, a bioluminescent compound, chemi luminescent compound, a metal chelator or an enzyme. A second molecule for conjugation to the antibody can be selected in accordance with the intended use. For example, for therapeutic use, the second molecule can be a toxin or therapeutic agent. Further, bi-specific antibodies specific for two or more epitopes may be generated using methods generally known in the art. Homodimeric antibodies may also be generated by cross-linking techniques known in the art (e.g., Wolff et al., Cancer Res. 53: 2560-2565).

### Pharmaceutical Compositions and Vaccines

The invention provides anti-idiotypic antibodies that are incorporated into pharmaceutical compositions, including immunogenic compositions (i.e., vaccines). Pharmaceutical compositions comprise one or more such compounds and, optionally, a physiologically acceptable carrier. Vaccines may comprise one or more such compounds and an adjuvant that serves as a non-specific immune response enhancer.

The adjuvant may be any substance that enhances an immune response to an exogenous antigen. Examples of adjuvants include conventional adjuvants, biodegradable microspheres (e.g., polylactic galactide), immunostimulatory oligonucleotides and liposomes (into which the compound is incorporated; see e.g., Fullerton, U.S. Patent No. 4,235,877). Vaccine preparation is generally described in, for example, M. F. Powell and M.J. Newman, eds., "Vaccine Design (the subunit and adjuvant approach)," Plenum Press (NY, 1995). Pharmaceutical compositions and vaccines within the scope of the present invention may also contain other compounds that may be biologically active or inactive. For example, one or more immunogenic portions of other tumor antigens may be present, either incorporated into a fusion polypeptide or as a separate compound, within the composition or vaccine.

A pharmaceutical composition or vaccine can contain DNA encoding one or more of the peptides as described herein, such that the polypeptide is generated in situ. As noted above, the DNA may be present within any of a variety of delivery systems known to those of ordinary skill in the art, including nucleic acid expression systems, bacteria and viral expression systems. Numerous gene delivery techniques are well known in the art, such as those described by Rolland, Crit. Rev. Therap. Drug Carrier Systems 15:143-198, 1998, and references cited therein. Appropriate nucleic acid expression systems contain the necessary DNA sequences for expression in the patient (such as a suitable promoter and terminating signal). Bacterial delivery systems involve the administration of a bacterium (such as Bacillus-Calmette-Guerrin) that expresses an immunogenic portion of the polypeptide on its cell surface or secretes such an epitope.

In a preferred embodiment, the DNA may be introduced using a viral expression system (e.g., vaccinia or other pox virus, retrovirus, or adenovirus), which may involve the use of a non-pathogenic (defective), replication competent virus. Suitable systems are disclosed, for example, in Fisher-Hoch et al., Proc. Natl. Acad. Sci. USA 86:317-321. 1989; Flexner et al., Ann. N. Y. Acad Sci. 569:86-103, 1989; Flexner et al., Vaccine 8:17-21, 1990; U.S. Patent Nos. 4,603,112, 4,769,330, and 5,017,487; WO 89/01973; U.S. Patent No. 4,777,127; GB 2,200,651; EP 0345242; WO 91/02805; Berkner-Biotechniques 6:616-B27, 1988; Rosenfeld et al., Science 252:431-434, 1991; Kolls et al., Proc. Natl. Acad. Sci. USA 91:215-219,1994; Kass-Eisler et al., Proc. Natl. Acad. Sci. USA 90:11498-11502, 1993; Guzman et al., Circulation 88:2838-2848, 1993; and Guzman et al., Cir. Res. 73:1202-1207, 1993. Techniques for incorporating DNA into such expression systems are well known to those of ordinary skill in the art. The DNA may also be "naked," as described, for example, in Ulmer et al., Science 259:1745-1749. 1993 and reviewed by Cohen, Science 259:1691-1692, 1993. The uptake of naked DNA may be increased by coating the DNA onto biodegradable beads, which are efficiently transported into the cells, and other means known to those skilled in the art.

While any suitable carrier known to those of ordinary skill in the art may be employed in the pharmaceutical compositions of this invention, the type of carrier will vary depending on the mode of administration. Compositions of the present invention may be formulated for any appropriate manner of administration, including for example, topical, oral, nasal, intravenous, intracranial, intraperitoneal, subcutaneous, intradermal or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (e.g., polylactate polyglycolate) may also be employed as carriers for the pharmaceutical compositions of this invention. Suitable biodegradable microspheres are disclosed, for example, in U.S. Patent Nos. 4,897,268 and 5,075,109.

In addition, the carrier may contain other pharmacologically-acceptable excipients for modifying or maintaining the pH, osmolarity, viscosity, clarity, color, sterility, stability, rate of dissolution, or odor of the formulation. Similarly, the carrier may contain still other pharmacologically-acceptable excipients for modifying or maintaining the stability, rate of dissolution, release, or absorption or penetration across the blood-brain barrier. Such excipients are those substances usually and customarily employed to formulate dosages for parenteral administration in either unit dose or multi-dose form or for direct infusion into the CSF by continuous or periodic infusion from an implanted pump.

Such compositions may also comprise buffers (e.g., neutral buffered saline or phosphate buffered saline), carbohydrates (e.g., glucose, mannose, sucrose or dextrans), mannitol, proteins, peptides or amino acids such as glycine, antioxidants, chelating agents such as EDTA or glutathione, adjuvants (e.g.. aluminum hydroxide) and/or preservatives. Alternatively, compositions of the present invention may be formulated as a lyophilizate. Compounds may also be encapsulated within liposomes using well known technology.

The compositions described herein may be administered as part of a sustained release formulation (i.e., a formulation such as a capsule or sponge that effects a slow release of compound following administration). Such formulations may generally be prepared using well known technology and administered by, for example, oral, rectal or subcutaneous implantation, or by implantation at the desired target site. Sustained-release formulations may contain a polypeptide, polynucleotide or antibody dispersed in a carrier matrix and/or contained within a reservoir surrounded by a rate controlling membrane. Carriers for use within such formulations are biocompatible, and may also be biodegradable; preferably the formulation provides a relatively constant level of active component release. The amount of active compound contained within a sustained release formulation depends upon the site of implantation, the rate and expected duration of release and the nature of the condition to be treated or prevented.

### Administration and Dosage

The compositions are administered in any suitable manner, often with pharmaceutically acceptable carriers. Suitable methods of administering cells in the context of the present invention to a subject are available, and, although more than one route can be used to administer a particular composition, a particular route can often provide a more immediate and more effective reaction than another route.

The dose administered to a patient, in the context of the present invention, should be sufficient to effect a beneficial therapeutic response in the patient over time, or to inhibit disease progression. Thus, the composition is administered to a subject in an amount sufficient to elicit an effective immune response to the specific antigens and/or to alleviate, reduce, cure or at least partially arrest symptoms and/or complications from the disease. An amount adequate to accomplish this is defined as a "therapeutically effective dose."

Routes and frequency of administration of the therapeutic compositions disclosed herein, as well as dosage, will vary from individual to individual, and may be readily established using standard techniques. In general, the pharmaceutical compositions and vaccines may be administered, by injection (e.g., intracutaneous, intratumoral, intramuscular, intravenous or subcutaneous), intranasally (e.g., by aspiration) or orally. Preferably, between 1 and 10 doses may be administered over a 52 week period. Preferably, 6 doses are administered, at intervals of 1 month, and booster vaccinations may be given periodically thereafter. Alternate protocols may be appropriate for individual patients. In one embodiment, 2 intradermal injections of the composition are administered 10 days apart.

A suitable dose is an amount of a compound that, when administered as described above, is capable of promoting an therapeutically relevant immune response, and is at least 10-50% above the basal (i.e., untreated) level. Such response can be monitored, for example, by measuring the anti-tumor antibodies in a patient or by vaccine-dependent generation of cytolytic effector cells capable of killing the target cells in vitro. Such vaccines should also be capable of causing an immune response that leads to an improved clinical outcome (e.g., more frequent remissions, complete or partial or longer disease-free survival) in vaccinated patients as compared to nonvaccinated patients. In general, for pharmaceutical compositions and vaccines comprising one or more peptides, the amount of each peptide present in a dose ranges from about 0.01 µg/kg to about 100 mg/kg body weight will be administered by intradermal, subcutaneous, or intravenous route. A preferred dosage is about 1 µg/kg to about 1 mg/kg, with about 5 µg/kg to about 200 µg/kg particularly preferred.

In general, an appropriate dosage and treatment regimen provides the active compound(s) in an amount sufficient to provide therapeutic and/or prophylactic benefit Such a response can be monitored by establishing an improved clinical outcome (e.g., more frequent remissions, complete or partial, or longer disease-free survival) in treated patients as compared to non-treated patients. Increases in preexisting immune responses to a protein generally correlate with an improved clinical outcome. Such immune responses may generally be evaluated using standard proliferation, cytotoxicity or cytokine assays, which may be performed using samples obtained from a patient before and after treatment.

The invention provides a method of delaying the onset of diabetes in a subject comprising administering to the subject a composition of the invention. Also provided is a method of inhibiting insulitis, or inducing an anti-idiotypic immune response, in a subject by administering such a composition to the subject. The subject can be a first degree relative of a Type 1 diabetes patient or otherwise be considered at increased risk of developing T1 D. In another embodiment, the subject is a newborn.

There are several levels of risk which would justify treatment with the anti-Id:
- genetic risk: HLA typing at birth. The risk is increased from 0.5% in the general population to 2% among those at genetic risk.
- genetic risk: HLA typing at birth. The risk is increased from 0.5% in the general population to 2% among those at genetic risk. - genetic risk among first degree relatives: the risk is increased from 0.5% to 3% if the mother has T1D, to 5% if the father has T1D, and to 8% if there is a sibling with T1D.
- a person with genetic risk who has developed any of the islet autoantibodies against GAD65, insulin, IA-2 or ZnT8. In subjects with GAD65Ab and two other antibodies, more than 50% of the subjects have developed T1D within 5 years.
- a person with genetic risk who has GAD65Ab and one other antibody: more than 25% develop T1D within 10 years.
- a person with genetic risk who has developed GAD65Ab only: more than 10% develop T1D within 10 years.

### Diagnostic Methods

Diagnostic methods for detecting Type 1 diabetes (T1D) and susceptibility to T1D and for detecting the presence of anti-idiotypic antibodies to GAD65 in a subject are described herein. The method comprises contacting a specimen with a molecule (such as an antibody) that specifically recognizes and binds the epitope of glutamate decarboxylase (GAD65) that is bound by antibody b96.11. The method further comprises detecting binding of the molecule to the specimen. The absence (or relative absence) of binding is indicative of susceptibility to Type 1 diabetes and of the absence of anti-idiotypic antibodies. In a typical embodiment, the molecule is an antibody. The antibody or other molecule can, optionally, be labeled with a detectable marker. Typically the specimen is obtained from a subject considered to be at increased risk for Type 1 diabetes, including a first degree relative (FDR) of a subject known to have T1D. Only 15% of all newly diagnosed T1D patients have a FDR with the disease. In one embodiment, the specimen is derived from a newborn. About 20% of all newborns could potentially benefit from being screened for GAD65 anti-Id, and offered treatment if their anti-ld is too low.

Detection of binding can be performed using conventional techniques known in the art, such as radioimmunoassay (RIA), western blot, immunoprecipitation, ELISA, and FACS analyses. Both competitive binding assays and direct detection assays can be used.

In one embodiment, the detection is by RIA. Details of an exemplary RIA are as follows. Serum samples (100[mu]I) are incubated with immobilized b96.11-Protein A Sepharose (25 [mu]I of 50% slurry) at 55[deg.]C for 10 min. The mixture is then incubated at 37[deg.]C for 30 min and at room temperature for a final 10 min and analyzed for GAD65Ab in RIA. The supernatant is separated from the beads by a two-step centrifugation. After the initial centrifugation step (5 min, 14,000 rpm) the supernatant is transferred to a new tube and centrifuged as before. The resultant supernatant is assayed for GAD65Ab in a RIA.

### Kits

Kits for use in diagnostic and therapeutic applications are described herein. Such kits can comprise a carrier, package or container that is compartmentalized to receive one or more containers such as vials, tubes, and the like, each of the container(s) comprising one of the separate elements to be used in the method, such as an anti-idiotypic antibody or composition of the invention.

The kit will typically comprise the container described above and one or more other containers comprising materials desirable from a commercial and user standpoint, including buffers, diluents, filters, needles, syringes, and package inserts with instructions for use. In addition, a label can be provided on the container to indicate that the composition is used for a specific therapeutic or non-therapeutic application, and can also indicate directions for either in vivo or in vitro use, such as those described above. Directions and or other information can also be included on an insert which is included with the kit.

In one embodiment, the kit comprises a container and at least one further container comprising antibody immobilized on a carrier, for example, using protein A-sepharose beads. The antibody can be a radiolabeled b96.11 antibody.

### EXAMPLES

The examples that follow are presented to illustrate the present invention and to assist one of ordinary skill in making and using the same. The examples are not intended in any way to otherwise limit the scope of the invention.

### Example 1: Modulation of diabetes in NOD mice by GAD65-specific monoclonal antibodies is epitope specific and accompanied by anti-idiotypic antibodies

This example shows that administration of a human monoclonal antibody specific to GAD65 (b96.11) to prediabetic NOD mice significantly delays the onset of autoimmune diabetes. This effect is epitope-specific as only b96.11 showed this therapeutic property, while a GAD65-specific human monoclonal control antibody (b78) derived from the same patient, but specific to a different determinant of GAD65, had no significant effect on the progression of disease. Administration of b96.11 or b78 to NOD mice was accompanied by the generation of anti-idiotypic antibodies. Importantly, the induced anti-idiotypic antibodies were specific for the immunizing antibody and blocked the binding of GAD65 by the respective antibody. These findings suggest a potential role for the internal image of the GAD65 determinant recognized by b96.11 in the anti-idiotypic antibody, supporting an immunomodulatory role. This example uses the following abbreviations: 65kDa isoform of glutamate decarboxylase: GAD65, autoantibodies to GAD65: GAD65Ab, Radioligand binding assay: RBA, Type 1 diabetes: T1D, intravenous immune globulin: IVIG.

### Materials and Methods

### Antibodies

Both GAD65-specific human monoclonal antibodies used in this study were derived from a patient with Autoimmune Polyendocrine Syndrome Type 2 ³⁶. B96.11 recognizes an epitope located at amino acid residues 308-365 ³⁶⁻³⁸, while b78 recognizes an epitope located at the C-terminus of GAD65 (512-540)³⁵⁻³⁶. Both antibodies are of the IgG1 subtype and their light chains are of the lambda subclass. The IgG show 80% similarities in the heavy chain and 79% similarities in the light chain, with the majority of differences located in the CDRs (Figure 1). The antibodies were purified from supernatants of the respective B cell line using Protein G Sepharose (Invitrogen, Carlsbad, CA). Polyclonal human IgG (The Binding Site, Birmingham, UK) does not contain GAD65 binding reactivity. All antibodies were dialyzed against PBS and sterile-filtered. The final concentration of the antibodies was 1 mg/ml in PBS.

### Radioligand binding assay (RBA)

Recombinant human ^{[35]}S-GAD65 was produced in an *in vitro* coupled transcription/translation system with SP6 RNA polymerase and nuclease treated rabbit reticulocyte lysate (Promega, Madison, Wl, USA) as described previously ³⁹. Briefly, animal sera (5µl) or IgG were incubated with ^{[35]}S-GAD65. After an overnight incubation at 4°C, antibody-bound ^{[35]}S-GAD65 was separated from unbound antigen with Protein A Sepharose (PAS) (Invitrogen) as a precipitating agent as previously described ⁴⁰. The immunoprecipitated radioactivity was counted on a Wallac Microbeta Liquid Scintillation Counter (Perkin Elmer Life and Analytical Sciences, Inc, Boston, MA). In competition-RBA GAD65-specific monoclonal antibody was incubated at its half-maximal binding concentration with serum from the injected animals. All samples were analyzed in triplicate determinations.

### Enzyme linked immunosorbent assay (ELISA)

**Detection of human antibodies:** Mouse sera were analyzed for the presence of human antibodies as follows: 96-well MAXI-SORP plates (Nalge Nunc International, Rochester, NY) were coated with goat anti-human antibodies (Bethyl Laboratories, Montgomery, TX) (1:100) overnight at 4°C. The plates were blocked with 1% BSA in PBS to reduce non-specific binding. Mouse serum was added to the wells and incubated for 2 hours at 37°C. Human antibodies were detected by 50 µl/well peroxidase labeled Goat anti-Human IgG (Bethyl Laboratories) (1:10,000) for 1 hour at 37°C. The plates were washed and incubated with the peroxidase substrate o-phenylenediamine dihydrochloride OPD) (Sigma-Aldrich Cooperation, St. Louis, MO). The reaction was stopped with 1M SulfuricAcid solution and the plates were read using a microplate reader at 450 nm. A standard curve consisting of human IgG dilutions was included on each assay.

**Detection of anti-idiotypic antibodies:** As above, but human recombinant Fab or human IgG at the indicated concentrations were used for the initial coating. Mouse serum were added to the wells and incubated for 2 hours at 37°C. Bound murine antibodies were detected with 50 µl (1:10,000) of peroxidase-conjugated goat anti-mouse IgG (Bethyl laboratories) per well. A standard curve for the determination of antibody levels (dilutions of goat anti-human IgG (Bethyl laboratories)) was generated for each assay. Negative controls consisted of mouse serum obtained from mice injected with PBS only.

### Mice

Female NOD mice were purchased at 3-4 weeks of age (Jackson Laboratories, Bar Harbor, ME). The mice were maintained in specific pathogen-free conditions in the animal facility at the University of Washington, Seattle. All animal experimentation was approved by the Animal Care and Use Committees of the University of Washington. The animals (groups of 8) were injected IP weekly with 10, 50, or 100 µg antibody or PBS. The injections started at five weeks of age and continued until the animals reached 35 weeks of age or developed diabetes. All animals were monitored for the development of diabetes. Hyperglycemia was determined by weekly weighing and blood glucose level tests. Blood glucose levels were measured with a Bayer Ascensia Elite meter and strips (Bayer HealthCare Diabetes Care, Tarrytown. NY) when the animal experienced a loss of 5-10% of body weight. Diabetes was defined by weight loss of 5-10% of body weight and blood glucose levels of >300 mg/dL for two consecutive weeks.

Upon confirmation of diabetes, the animal was sedated with ketamine/xylazine and killed by heart puncture. The pancreas was perfusion-fixed in 4% paraformaldehyde and embedded in paraffin wax. Sections of 5-µm were mounted on glass slides and stained with hematoxylin and eosin for histological analysis.

### Insulitis scoring

A minimum of 41 islets/group were scored for insulitis. Scoring was performed under double-blinded conditions. The degree of insulitis was graded according to the following: normal islet: (score 1), perivascular/periductal infiltration (score 2), peri-insulitis (score 3), mild insulitis (<25% of the islet infiltrated; score 4), and severe insulitis (more than 25% of the islet infiltrated, score 5).

### Statistical analysis

The control animals injected with PBS or polyclonal human IgG were combined into one group, because no difference in incidence rate, age at disease onset, or degree of insulitis was detectable. Similarly, groups injected with b78 IgG (50 and 100 µg) showed no difference in incidence, age at disease onset, or degree of insulitis and were therefore combined into one group.

Weight gain in the different animal groups was compared using Mann-Whitney U.

Differences in the incidence rates in the different treatment groups were compared using Chi-squared test with 1 degree of freedom. Fisher's exact test, two sided, and permutation test, two sided. All analyses indicated a significant difference between the control group and animals injected with 100 µg b96.11. Reported here are the results of the most conservative analysis, namely chi-squared test with one degree of freedom.

### Results

### Injections with b96.11 delay the onset and reduce the incidence rate of diabetes

Animals were injected with GAD65-specific monoclonal Ab, human polyclonal antibody, or PBS. The animals were monitored weekly for diabetes development. Animals injected with PBS or human polyclonal IgG did not differ in their incidence rate of diabetes and the data obtained from these animals was combined. The mice started to develop diabetes at seven weeks of age. Nineteen/24 (80%) animals developed diabetes by week 28. Injections with 50 and 100 µg b78 IgG yielded a cumulative incidence rate of 50%, and 38%, respectively, with no significant difference between the two dosage groups or from the control animals.

Injections with 50 and 100 µg b96.11 resulted in a cumulative incidence rate of 25% (2/8). The two animals in the lower dose group developed diabetes at week 15, the first animal in the 100 µg group developed diabetes at week 18 and the second animal at week 33 (Figure 2). Only injections with 100 µg b96.11 IgG resulted in significant reduction in incidence rate and delay in onset, as compared to the control animals (p=0.006). Injections with b96.11 at the lower dosage showed only a trend towards prevention, suggesting that the effect was dose-dependent, injections with 10 µg b96.11 induced no reduction in incidence or delay in onset. The injections with 100 µg b96.11 IgG were repeated once more with similar results.

### GAD65Ab b96.11 reduces the severity of insulitis

The above effect was also reflected in the severity of insulitis. Insulitis was less severe in animals treated with 100 and 50 µg b96.11 IgG as compared to the control group and animals injected with b78 IgG (Table 1 and Figure 3). Insulitis severity showed a dose response to injected b96.11 IgG.

**Table 1: Degree of insulitis in NOD mice**

| Score | 1 | 2 | 3 | 4 | 5 |
|---|---|---|---|---|---|
| b36.11 100µg | 64 | 28 | 9 | 0 | 0 |
| b96.11 50µg | 54 | 29 | 15 | 2 | 0 |
| b96.11 10µg | 39 | 39 | 13 | 10 | 0 |
| b78 | 17 | 34 | 32 | 15 | 2 |
| controls | 22 | 19 | 15 | 24 | 19 |

Islets obtained from animals injected with b96.11 IgG (100. 50, and 10 µg), b78 IgG, and controls were scored as normal islets (score 1), perivascular/periductal infiltration (score 2), peri-insulitis (score 3), mild insulitis (<25% of the islet infiltrated; score 4), and severe insulitis (more than 25% of the islet infiltrated, score 5). The mean score for each group was calculated by dividing the total score by the number of islets scored.

### Weight

The animals were weighed weekly to monitor their growth and potential weight loss as an early sign of diabetes. The last two weight measurements before euthanasia were removed, because the animals were terminated after two weeks of consecutive hyperglycemia and weight loss. The longitudinal weight measurements between groups were not significantly different.

### Presence of human IgG in mouse sera

Blood samples derived from the animals two weeks after the final injection were tested for the presence of human IgG by ELISA. Significant presence of human IgG was found in all animals, except in mice injected with PBS only amino acid residues 308-365. The human IgG titer in animals injected with 100 µg IgG was twice as high (median of 200 µg/ml) than that observed in animals injected with 50 µg (median of 100 µg/ml) and 5-times higher than in animals injected with 10 µg IgG (median of 40 µg/ml).

However, reactivity to GAD65 could not be detected in the mouse sera, with the exception of two sera, both obtained from animals injected with 10 µg of b96.11.

### Presence of anti-idiotypic antibodies

Because the mice were injected with human IgG, a human-specific immune response in the animals was anticipated. ELISA analysis of the mouse sera revealed the presence of anti-idiotypic antibodies specific to human Fab in all animals injected with human IgG. The levels of the anti-idiotypic antibodies correlated with the dosage of injected IgG. The median concentration of anti-idiotypic antibodies in animals injected with 50 or 100 µg IgG was significantly higher compared with animals injected with 10µg IgG (p=0.0002). No difference in the anti-idiotypic antibody levels was found when comparing animals injected with different IgG.

### Anti-idiotypic antibodies prevent binding of GAD65-specific monoclonal antibodies to GAD65

The binding specificities of the anti-idiotypic antibodies was investigated by testing whether they interfered with the binding of b96.11 and b78 to GAD65. In competition-RBA the respective GAD65-specific monoclonal antibody was incubated at its half-maximal binding concentration with serum from the injected animals (Figure 4a). Sera from b96.11-injected mice inhibited the binding of b96.11 IgG and Fab to GAD65, but not the binding of b78 IgG or Fab. Likewise, sera from b78-injected animals specifically inhibited the binding of b78 IgG and Fab to GAD65, but not that of b96.11 IgG or Fab (Figure 4b). Sera obtained from animals injected with human polyclonal antibody, or PBS, had no effect on the binding capacity of b96.11 or b78.

The level of anti-idiotypic antibodies specific to the antigen-binding site of the GAD65Ab depended on the dosage of injected IgG. Animals injected with 100 µg b96.11 had significantly higher levels of anti-idiotypic antibodies interfering with GAD65 binding, than animals in the 10 µg group (p=0.04).

### Discussion

T1D-specific monoclonal GAD65Ab b96.11 significantly reduced diabetes development in NOD mice. This effect was both antigen-, epitope-, and dose- specific, since neither administration of polyclonal human IgG nor that of GAD65-specific monoclonal antibody b78 had an effect on the development of diabetes, and only the highest dosage of b96.11 resulted in significant reduction.

Injections with human antibodies induced the formation of anti-idiotypic antibodies in the animals, some of which recognized the antigen-binding site of the monoclonal antibodies and consequently interfered with the binding to GAD65. These anti-idiotypic antibodies likely present internal images of GAD65 bound by b96.11 and thus may mimic GAD65. Alternatively, the internalized GAD65/b96.11 complexes could induce a modulation of GAD65 processing, thus modifying antigen presentation.

The two GAD65-specific monoclonal antibodies were generated from the same individual but recognize different epitopes. B96.11 shares epitope specificities with GAD65Ab present in sera obtained from T1 D patients ^{32, 33}, and in a longitudinal study of healthy GAD65Ab-positive schoolchildren it was shown that the specificity to the b96.11-defined epitope increases with time in high risk children only ³⁴. GAD65Ab with epitopes similar to that recognized by b78 are not common in T1D patients, but may be observed in sera from Stiff Person Syndrome (SPS) patients, where this antibody specificity correlates with the ability of the sera to inhibit GAD65 enzymatic activity ³⁵.

In mice, human monoclonal antibodies and their antigen complexes are most likely taken up via pinocytosis, rather than via Fc-Receptor binding. Earlier reports showed that human IgG injected IP to mice was eliminated from the animals with a half life of 2-3 days ⁴¹. It is therefore surprising that human IgG was detected in circulation after two weeks. Moreover, the level of human IgG in circulation exceeds that of the injected bolus, suggesting that the IgG accumulates in the animals. The mechanism by which the antibodies are retained in the animals and the location of the antibody other than in blood can be further investigated. The initial efforts to localize the antibody in the animals were restricted to the pancreas. Significant amounts of human antibody were not found in the islet or elsewhere in the pancreas.

The injection with human IgG triggered the development of significant amounts of anti-idiotypic antibodies in the animals, some of which inhibited the binding of GAD65 by the respective monoclonal antibody or its Fab. The data presented here suggests that the anti-idiotypic antibodies recognize the GAD65-binding domain of their respective monoclonal antibodies, thus serving as an internal-image antibody. The anti-idiotypic antibodies may mimic GAD65-administration as the internal image binds to the idiotypic antibodies. Monoclonal anti-idiotypic antibodies specific to b96.11 can be developed to confirm that these anti-idiotypic antibodies interfere with the pathogenesis of T1D.

The injection of b96.11 monoclonal antibodies may also affect the processing and presentation of GAD65, as was suggested in earlier studies. Antibody-induced modulation of antigen processing may affect the presentation of the antigen to auto-reactive T cells. Experiments to test changes in GAD65-specific T cell responses in the NOD mice are necessary to test this hypothesis.

The role of anti-idiotypic antibodies in the regulation of the immune system was suggested first in the network hypothesis by Jeme ⁴² proposing that idio-types and anti-idiotypes create a homeostasis of the adaptive immune response. The presence of anti-idiotypic antibodies has been described for a number of autoantibodies ⁴³ and autoimmune diseases (for review see ⁴⁴). Anti-idiotypic antibodies may bind the antigen-binding site of the antibody and thus represent the internal image of the antigen. Supporting this hypothesis is the finding that the anti-idiotypic antibody serum levels correlate inversely with that of autoantibodies in several autoimmune diseases ⁴⁵⁻⁴⁷. The presence of anti-idiotypic antibodies in healthy individuals that successfully block the binding of pathogenic autoantibodies has been demonstrated suggesting that these anti-idiotypic antibodies regulate potential pathogenic autoantibodies ^{48, 49}. Moreover, the successful treatment of autoimmune disorders with intravenous immune globulin (IVIG) has partially been explained by the presence of anti-idiotypic antibodies (for review see ^{50; 51}). IVIG treatment in T1D patients was administered with different results. In new onset T1D patients the treatment showed improvement in regard to C-peptide levels and two out of eight patients ceased to require insulin treatment IVIG treatment in T1 D patients was administered with different results. In new onset T1D patients the treatment showed improvement in regard to C-peptide levels and two out of eight patients ceased to require insulin treatment ⁵². In established T1D patients, IVIG did not show a change in remission frequency or beta cell function ⁵³. Recent studies suggest that the successful treatment of SPS patients with IVIG induces a reduction in GAD65Ab titers ⁵⁴. Moreover, insulin-mimicking anti-idiotypic antibodies have been described in the development of type 1 diabetes in another animal model of the disease, namely the BioBreeding rat (BB rat) ⁵⁵.

### References cited in Example 1:

1. Shoda LK, et al. Immunity 2005; 23: 115-26.
2. Bach J-F. Endocrine Rev. 1994; 15: 516-42.
3. Singh B, Rabinovitch A. Autoimmunity 1993; 15: 209-13.
4. Ohsugi T, Kurosawa T. Lab Anim Sci 1994; 44: 386-8.
5. Haskins K. Adv Immunol 2005; 87: 123-62.
6. Wong FS, Wen L. Rev Diabet Stud 2005; 2: 121-35.
7. Serreze DV, et al. J. Exp Med 1996; 184: 2049-53.
8. Atkinson MA, Leiter EH. Nat Med 1999; 5: 601-4.
9. Bach JF. Arthritis Res 2002; 4: S3-15.
10. Maki T, et al. Proc Natl Acad Sci U S A 1992; 89: 3434-8.
11. Makhlouf L, et al. Transplantation 2004; 77: 990-7.
12. Herold KC. et al. N Engl J Med 2002; 346: 1692-8.
13. Kaufman DL, et al. Nature 1993; 366: 69-72.
14. Ramiya VK, et al. J Autoimmun 1997; 10: 287-92.
15. Jun H-S, et al. Diabetologia 2002; 45: 668-76.
16. Petersen JS, et al. Diabetes 1994; 43: 1478-84.
17. Tisch R, et al. J Autoimmun 1994; 7: 845-50.
18. Kim J, et al. Diabetes 1993; 42: 1799-808.
19. Tisch R, et al. Diabetes 1998; 47: 894-9.
20. Tisch R, et al. J Immunol 1999; 163: 1178-87.
21. Weaver DJ, Jr., et al. J Immunol 2001; 167: 586-92.
22. Bot A, et al. J Immunol 2001; 167: 2950-5.
23. Tisch R, et al. J Immunol 2001; 166: 2122-32.
24. Cetkovic-Cvrlje M, et al. Diabetes 1997; 46: 1975-82.
25. Ziegler B, et al. Diabetes Res 1994; 25: 47-64.
26. Velloso LA, et al. Clin Exp Immunol 1994; 96: 129-37.
27. Bonifacio E, et al. Diabetes 2001; 50: 2451-8.
28. Jaume JC, et al. J Immunol 2002; 169: 665-72.
29. Banga JP, et al. Clin Exp Immunol 2004; 135: 74-84.
30. Reijonen H, et al. Diabetes 2000; 49: 1621-6.
31. Menard V, et al. Pharm Res 1999; 16: 1059-66.
32. Padoa CJ, et al. Diabetes 2003; 52: 2689-95.
33. Gilliam LK, et al. Clin Exp Immunol 2004; 138: 337-41.
34. Schlosser M, et al. Diabetologia 2005; 48: 922-30.
35. Raju R, et al. J Immunol 2005; 175: 7755-62.
36. Tremble J, et al. J Clin Endocrinol Metab 1997; 82: 2664-70.
37. Schwartz HL, et al. J Mol Biol 1999; 287: 983-99.
38. Fenalti G, et al. Mol Immunol 2007; 44: 1178-89.
39. Grubin CE, et al. Diabetologia 1994; 37: 344-50.
40. Falorni A, et al s. J Immunol Methods 1995; 186: 89-99.
41. Peterson JW, et al. Infect Immun 2006; 74: 1016-24.
42. Jerne NK. Ann Immunol (Paris) 1974; 125C: 373-89.
43. Stea EA, et al. Arthritis Rheum 2006; 54: 2228-34.
44. Rossi F, et al. Immunol Rev 1989; 110: 135-49.
45. Silvestris F, et al. Arthritis Rheum 1984; 27: 1387-96.
46. Jayne DR, et al. J Autoimmun 1993; 6: 221-6.
47. Rossi F, et al. Clin Exp Immunol 1991; 83: 298-303.
48. Escher R. et al. Br J Haematol 1998; 102: 820-8.
49. Escher R, et al. J Autoimmun 2002; 18: 71-81.
50. Kazatchkine MD, Kaveri SV. N Engl J Med 2001; 345: 747-55.
51. Sapir T, Shoenfeld Y. Clin Rev Allergy Immunol 2005; 29: 185-99.
52. Panto F, et al. J Autoimmun 1990; 3: 587-92.
53. Colagiuri S, et al.. Clin Exp Rheumatol 1996; 94: S93-7.
54. Dalakas MC. J Neurol 2005; 252: I19-25.
55. Elias D, et al. Diabetes 1990; 39: 1467-71.

### Example 2: Lack of anti-idiotypic antibodies, not presence of the corresponding autoantibodies to glutamate decarboxylase, defines type 1 diabetes

This example investigates the presence of GAD65Ab in healthy individuals (n=238), and first degree relatives (FDR) of T1 D patients (n=27) who tested negative for GAD65Ab in conventional RIAs. Sera were applied to affinity columns coated with GAD65-specific monoclonal antibodies to absorb anti-idiotypic antibodies (anti-Id). The absorbed sera were analyzed for binding to GAD65 by RIAs. Both healthy individuals and FDR present GAD65Ab that are inhibited by anti-Id, masking them in conventional detection methods. The presence of GAD65Ab-specific anti-Id was confirmed by competitive ELISA. Remarkably, T1D patients (n=54) and Stiff Person Syndrome (SPS) patients (n=8) show a specific lack of anti-Id to disease-associated GAD65Ab epitopes. Purified anti-Id from healthy individuals and FDR inhibited the binding of GAD65Ab from T1D patients to GAD65. This example shows that masked GAD65Ab are present in the healthy population and that a lack of particular anti-Id, rather than GAD65Ab per se, is a characteristic of T1 D. The lack of these inhibitory antibodies may contribute to T cell activation by GAD65Ab.

### Materials and Methods

### Serum samples

Healthy GAD65Ab-negative FDR of T1D patients (n = 27) (mean age 39 years, range 17-61 years) were identified by screening individuals from families with at least two siblings with diabetes to a total of 1170 probands with T1D enrolled in the Diabetes Incidence Study in Sweden and the Swedish Childhood Diabetes registry. The samples in this study were chosen at random from the GAD65Ab-negative sera. Sera of healthy individuals (n=238) (15-34 years old) were randomly selected control samples matched to newly diagnosed T1 D patients. These samples were collected in 1992-1993 in the Diabetes Incidence Study in Sweden. All serum samples tested repeatedly negative for antibodies to GAD65 in conventional RIA.

GAD65Ab-positive T1D patients (n=54) were randomly selected from 15-35 year old newly diagnosed Swedish T1D patients. These patients were registered in 1992-1993 in the Diabetes Incidence Study in Sweden and were previously reported to be positive for GAD65Ab.

GAD65Ab-positive SPS patients (n=8) were collected in Seattle from 6 females and 1 male. Another male SPS patient was evaluated at King's College Hospital (London, U.K.). All subjects or their legal guardians gave informed consent.

Local institutional ethics committee approval and subjects' consent was obtained prior to collection of all serum samples.

### Antibodies

Human monoclonal antibodies b96.11 and b78 specific to GAD65 were derived from a patient with autoimmune polyendocrine syndrome type 2 (46). B96.11 recognizes an epitope that is specifically bound by patients with T1D (13). B78 recognizes an epitope that is specifically bound by patients with SPS (14). The epitope specificities of these antibodies are described in detail in a recent publication (36). Briefly, they recognize epitopes located at independent clusters that are positioned on opposing faces of the molecule's C-terminal domain. HAA1 is a human monoclonal antibody (ATCC, Manassas, VA) specific to blood group A antigen (47) and shows no binding to GAD65. The antibodies were purified from supernatants of the respective B cell line using Protein G Sepharose (Invitrogen, Carlsbad, CA).

### Radioligand binding assay

GAD65Ab were determined using the RIA previously described (48). Briefly, recombinant [35]S-GAD65 was produced in an in vitro coupled transcription and translation system with SP6 RNA polymerase and nuclease treated rabbit reticulocyte lysate (Promega, Madison, WI). Sera or IgG at the indicated concentrations were incubated with [35]S-GAD65 (25,000 of TCA precipitable radioactivity). Sera were initially analyzed at 2.5%l. If the GAD65Ab titer exceeded 9000 cpm, the sample was diluted and re-analyzed. The reported titer was adjusted accordingly. After an overnight incubation at 4°C, antibody-bound [35]S-GAD65 was separated from unbound antigen by precipitation with Protein A-Sepharose (PAS) (Invitrogen). The immunoprecipitated radioactivity was counted on a Wallac Microbeta Liquid Scintillation Counter (Perkin Elmer Life and Analytical Sciences, Inc, Boston, MA).

### Western blot analysis

Protein samples were electrophoresed on 15% SDS-PAGE under reducing conditions. Proteins were electrotransferred from the gel to lmmobilon-P membranes (Millipore, Boston, MA). Membranes were blocked in TBS buffer containing 1% BSA and incubated with goat anti-human kappa light chain-HRP (AbD Serotec, Raleigh, NC), goat anti-human lambda light chain-HRP (Serotec), goat anti human IgG-HRP (Bethyl Laboratories, Montgomery, TX), or mouse anti-human IgM-HRP (Invitrogen Corporation, Carlsbad, CA) at the concentration recommended by the manufacturer. The immunocomplexes were detected with Enhanced Chemiluminescence Detection system (GE Healthcare. Piscataway, NJ).

### ELISA

Binding of anti-Id to GAD65Ab b96.11 and b78 was measured by ELISA following standard methods. Anti-Id were isolated from a healthy individual and absorbed to 96-well microtiter plates (Nunc Maxisorb, Roskilde, Denmark) (50µl/well), The antibodies were detected by purified b96.11 and b78 that had been labeled with HRP according to the manufacturer's instructions (Pierce Biotechnology, Rockford, IL). Binding was competed by recombinant human GAD65 (Diamyd Medical AB, Sweden) or BSA.

### Crosslinking of antibody to PAS

Purified antibodies were crosslinked to PAS using the Dimethylpimelimidate method (49). One mg of antibody was crosslinked to one ml of PAS beads (Invitrogen). The efficiency of the coupling was 50% (0.5mg/ml). There was no significant release of the coupled protein from PAS as tested by RIA and Western blot analysis.

### Absorption on antibody-coupled PAS beads.

Serum samples (100µl) were incubated with antibody-PAS beads (25 µl of 50% slurry) for 1 hour. The bead volume was previously titrated for optimal assay conditions. The bound fraction was separated from the unbound serum by gravity flow. The flow-through of the column was analyzed for GAD65Ab in a RIA. Change in volume was accounted for.

### Heat dissociation

To dissociate complexes of GAD65Ab and their bound inhibitors in serum samples, the heat dissociation method as described (15) was followed. Briefly, samples (100µl) were heated to 55°C for 10 min in the absence or presence of antibody-PAS beads (25 µl of 50% slurry). The mixture was then incubated at 37°C for 30 min and at room temperature for a final 10 min and analyzed for GAD65Ab in RIA. In absorption experiments the flow-through was assayed for GAD65Ab in a RIA.

### Competition experiment

To evaluate the antibody binding specificity, 150 nmol/l unlabelled recombinant human GAD65 (Diamyd Medical AB, Sweden) was used to block the binding of ⁽³⁵⁾S-GAD65 to GAD65Ab. Unlabelled 150 nmol/l BSA was used as a control.

### Elution of bound inhibitor

Bound protein was eluted from b96.11-PAS with 0.05M Glycine buffer (pH 11). One ml fractions were collected and the pH was immediately adjusted to pH 7.

### Preparation of Ig and Ig-depleted fractions of sera.

Ig was purified from sera by successive affinity chromatography on PAS and PGS (Invitrogen). Western blot analysis confirmed the presence of Ig in the purified fraction, while the Ig-depleted fraction contained no Ig.

### Statistical analysis

All samples were analyzed in duplicate determinations. The mean intra-assay coefficient of variation was 5% (13-0.04%). The assay for GAD65Ab showed good sensitivity (86%) and high specificity (93%) in the 2007 Diabetes Antibody Standardization Program Workshop. Positive and negative controls were included for each assay. Binding levels between different treatments within one serum group were adjusted to the untreated positive and negative controls to correct for inter-assay variations. Binding levels between different serum groups were adjusted to the same serum derived from a healthy individual that was included on each plate and underwent identical procedures with the other samples. Median GAD65Ab levels between groups were analyzed using the non-parametric analysis of variance (Kruskall-Wallis test) followed by Dunn's multiple comparisons test. Significance was defined by P<0.05.

### Results

### Sera from healthy individuals and FDR contain GAD65Ab that are masked, while T1D patients and SPS patients lack inhibitors to disease-specific GAD65Ab.

The presence of masked GAD65Ab was investigated in human serum samples. Samples were incubated at 55°C either in the presence or absence of monoclonal GAD65Ab immobilized to protein A Sepharose (PAS). After the reaction was allowed to cool to room temperature the flow-through was tested in a RIA for binding to GAD65 (Figure 1a). Sera of healthy individuals (n=238) and FDR (n=27) that had tested negative for GAD65Ab in conventional RIAs, GAD65Ab-positive T1D patients (n=54) and GAD65Ab-positive SPS patients (n=8) were analyzed.

For the healthy individuals a significant increase in binding to GAD65 was observed after absorption to b96.11-PAS or b78-PAS (median binding before absorption: 292 (112-1055) cpm, after absorption to b96.11-PAS: 1088 (401-8881) cpm, after absorption to b78-PAS: 6519 (2056-16278) cpm) (p<(3.001). The GAD65Ab titer of all samples after absorption to both b96.11-PAS and b78-PAS was higher than the median before absorption and 123/238 (52%) of the b96.11-absorbed samples had a GAD65Ab titer above the maximum GAD65Ab titer before absorption. All samples absorbed to b78-PAS tested above the maximum GAD65Ab titer before absorption.

Similar results were obtained when absorbing the sera without previous heat-dissociation. However, the effect was less pronounced, indicating that the moderate temperature increase catalyzed the dissociation of the GAD65Ab/***inhibitor*** complexes and allowed the absorption of the released ***inhibitor*** to the immobilized GAD65Ab. Dissociation of antibody complexes by heat has been employed previously (15) and the results obtained confirmed that the binding capacity of purified GAD65Ab remains stable under these conditions.

Binding of the isolated inhibitor to GAD65Ab was confirmed in an ELISA (Figure 5B). The inhibitor was eluted from b96.11-PAS or b78-PAS after absorption of serum obtained from a healthy individual and detected by HPR-labeled b96.11 or b78, respectively. Addition of human recombinant GAD65 diminished the signal in a dose-dependent manner, suggesting that the inhibitor binds to the antigen-binding site of GAD65Ab. Addition of BSA had no effect on the binding.

A significant increase in GAD65Ab levels after absorption to b96.11-PAS and b78-PAS was also observed for FDR (p<0.001) (Figure 5A). In the analysis of the T1D patients, a significant increase from 1094 to 9830 cpm in the level of GAD65Ab was observed after absorption on b78-PAS (p<0.001), however no difference was detected between the level of GAD65Ab in non-absorbed and b96.11-PAS absorbed sera. In the analysis of the SPS patients no increase in the level of GAD65Ab was found following absorption on either b96.11-PAS or b78-PAS.

The possible release of GAD65Ab from the GAD65Ab-PAS was tested by analyzing the flow-through of GAD65Ab-PAS that underwent the same procedure without incubation with serum. No binding to GAD65 could be detected under these conditions.

The observed binding was specific to GAD65, since competition with unlabeled human recombinant GAD65, but not BSA reduced the binding to radiolabeled GAD65 (p<0.001) (Figure 6A). Moreover, no increase in binding to the tyrosine phosphatase like protein IA-2 was observed when testing sera that had been heated and absorbed.

Whether the effect was specific to GAD65Ab was investigated by absorbing serum samples to an irrelevant human monoclonal antibody HAA1 immobilized to PAS. This antibody recognizes blood group A antigen. Fourteen healthy individuals and 14 FDR were tested. No significant increase in GAD65Ab binding was observed (Figure 6B).

### Characterization of the inhibitor

The inhibitor was identified as human Ig by preparing purified Ig and Ig-depleted fractions from sera of four FDR. The purified Ig and the Ig-depleted sera were incubated with b96.11-PAS to identify the fraction that contained the inhibitor and GAD65Ab (Figure 7A). As expected, absorption of the purified Ig fraction on b96.11-PAS resulted in a significant increase in binding to GAD65, while absorption of the Ig-depleted fraction on b96.11-PAS had no effect on GAD65 binding. These data suggest that 1) the immunoglobulin fraction contains both GAD65Ab and the inhibitor, and 2) that the observed effect is not due to release of b96.11 from b96.11-PAS by serum proteases.

The inhibiting Ig was further analyzed by eluting the serum fraction absorbed to b96.11-PAS and testing the eluted fractions for the presence of human Ig by Western blot analysis (Figure 7B). In addition to both human kappa and lambda light chains, Western blot analysis showed the inhibiting fractions to contain human IgG, while testing with human IgM showed no signal. As an additional control, no signal was found in Western blot analysis using fractions eluted from b96.11-PAS beads that had not been incubated with human serum, indicating that the coupled b96.11 was not released from the b96.11-PAS.

These results led to characterization of the inhibitor as anti-Id.

### Anti-Id show cross-reactivity to GAD65Ab from different individuals

Anti-Id were eluted from b96.11-PAS after absorption with serum from a FDR as described above. Dose-dependent inhibition of GAD65 binding was observed when the anti-Id was added to the absorbed sample (Figure 8A). Following the same protocol, anti-Id was prepared from a healthy individual and carried out cross-inhibition experiments between the groups using the optimal conditions (20µl) as determined above (Figure 8B). The data suggest that anti-Id isolated from healthy individuals efficiently inhibit antigen binding by absorbed sera from FDR and vice versa. Moreover, anti-Id obtained from a healthy individual and a FDR also inhibited GAD65 binding by four T1D patients' sera (Figure 8B), although this inhibition was weaker (74 and 79%, respectively) as compared to that in the healthy individual and the FDR (∼90%).

### Discussion

This example demonstrates for the first time anti-Id specific to GAD65Ab. Immune depletion of these anti-Id reveals the presence of masked GAD65Ab in the majority of sera from healthy individuals and FDR that otherwise test negative for GAD65Ab in conventional screening assays. Moreover, the data presented here show that GAD65Ab-specific anti-Id are severely reduced in T1D patients. In particular, anti-Id reactive to mAb b96.11 that is associated with progression to T1D (13) show a marked reduction in T1D patients. The study suggests that GAD65Ab are found in the majority of healthy, non-diabetic individuals, but that their binding capacity is inhibited by GAD65Ab-specific anfi-id. It is the absence of these anti-Id, rather than the presence of GAD65Ab, that is characteristic for T1 D. Screening of sera for anti-Id specific to disease-associated GAD65Ab provides a valuable tool in the characterization and risk assessment for T1 D.

Anti-Id have been described in the normal human immune response, where they are believed to play a protective role, as they can block the binding of pathogenic autoantibodies (16). They have also been described in several autoimmune diseases (17-20). Autoantibodies to ribosomal P proteins (anti-P) are regarded as specific for patients with systemic lupus erythematosus. Stafford and colleagues demonstrated that in healthy individuals who tested negative for anti-P antibodies by conventional serologic screenings, anti-P antibodies were in fact readily detectable after removal of the inhibitory antibodies (21-23). Similarly, autoantibodies to the E2 subunit of the pyruvate dehydrogenase complex, thought to be specific to patients with primary biliary cirrhosis, are present but inhibited by anti-Id in healthy individuals (24). In T1D, anti-Id to insulin autoantibodies have been reported both in humans (25) and in the BB rat model (26). These anti-Id appear to resemble insulin and may affect the utilization of insulin (27). A balance between autoantibodies and anti-Id is proposed to contribute to the homeostasis of the adaptive immune response in the "network Hypothesis" (28). A disturbance of this balance - by a decline in protective factors or an increase in autoimmune elements - could precipitate autoimmune disease. GAD65Ab-specific anti-Id are likely formed in the following cascade of events: death of pancreatic beta cells leads to the release of islet cell antigens, including GAD65. This beta cell death can be the result of a beta cell injury or apoptosis during normal development (29, 30). The islet cell antigens are presented in the pancreatic lymph nodes (31) and lead to the formation of GAD65Ab. Indeed, the presence of a wide range of autoantibodies - including GAD65Ab-shortly after birth has been described (32). According to the Network Hypothesis antibody idiotypes will induce the production of anti-Id (28, 33). These anti-Id can then block the initial antibodies, preventing them from binding to the antigen.

However the actual mechanisms involved in the lack of anti-Id in autoimmune diseases have not been well studied, and most studies of anti-Id address autoimmune diseases with a major B cell component to the immune response, whereas in T1D, although autoantibodies are readily demonstrable, the pathogenesis is considered to be T cell mediated.

In the light of recent evidence that autoantibodies may play a role in modulating the T cell response (11), it is notable that antibody specificity, rather than antibody titer, is associated with T1D and disease progression (13, 34, 35). The GAD65Ab response in T1D patients is characterized by GAD65Ab specificities similar to that of monoclonal antibody b96.11 (13), while GAD65Ab in patients with SPS often recognize GAD65Ab epitopes similar to that bound by b78 (14). These two antibodies recognize epitopes located at independent clusters (ctc1 and ctc2), positioned on opposing faces of the C-terminal domain of the GAD65 molecule (36). Recognition of ctc2 by b96.11-like GAD65Ab appears to be associated with an aggressive islet cell autoimmune reaction leading to T1D, while binding to the ctc1 epitope by b78-like GAD65Ab appears to be characteristic of an islet cell autoimmunity either unassociated with diabetes (e.g. SPS), or with a milder diabetes phenotype. Similarly, it is a lack of anti-Id specific to b96.11, and not b78, that is associated with T1D.

The lack of anti-Id specific to b96.11 in T1D patients may suggest that GAD65Ab are functionally inhibited by epitope-specific anti-Id in the healthy immune system. In the absence of these specific inhibitory anti-Id, overt GAD65Ab are exposed and consequently are able to modulate the immune response (8-10) and break T cell tolerance as recently demonstrated (11). Perhaps antibodies to ctc2 epitope(s) play a role in antibody-driven effector T cell activation in T1 D equivalent to the cooperative role played by antibodies to ovalbumin in the development of diabetes by T cells in RIP-mOVA mice (11) and such activation is blocked by the presence of anti-Id of appropriate specificity.

While anti-Id are used in the treatment of allergy (for review see (37)), they are currently not used in the treatment of autoimmune diseases. However, the successful treatment of autoimmune disorders with intravenous immune globulin has partially been explained by the presence of anti-Id (for review see (38, 39)), and the potential of recombinant anti-Id has been suggested in the treatment of some autoimmune diseases (40). The rationale for using GAD65Ab-specific anti-Id in treatment is to provide enough anti-Id immunoglobulins to neutralize circulating GAD65Ab of the b96.11-idiotype.

The presence of anti-Id may provide a mechanism for some previously unexplained phenomena. For example, these GAD65Ab-specific anti-Id may explain the persistence of GAD65Ab for years after the onset of T1D (41, 42), despite the loss of beta cells as the antigen source after disease onset Continuous beta cell regeneration, protein mimicry, incomplete destruction of the beta cells, release of GAD65 from other sources, or crossreactivity of GAD65Ab with other proteins have also been proposed to explain this phenomenon (43). An antigen-independent memory likely could be generated by the interaction of GAD65Ab-specific B cells with complementary B cells that produce anti-Id (for review see (44)). Moreover, the anti-Id explain previous evidence of a serum factor preventing the binding of autoantibodies to GAD65 observed by Brown & Christie (45).

In conclusion, the findings of this example demonstrate that GAD65Ab are not confined to T1D, but are masked in healthy individuals by specific anti-Id that are absent in T1 D. Anti-Id may play a protective role in the immune response, by preventing GAD65Ab to bind to their antigen and potentially modulate T cell responses to GAD65.

### References cited in Example 2:

1. Baekkeskov, S., et al. (1990) Nature 347, 151-6.
2. Falorni, A. & Brozzetti, A. (2005) Best Pract Res Clin Endocrinol Metab 19, 119-33.
3. Falorni, A., et al. (2002) Endocrinol Metab Clin North Am 31, 369-89 vii.
4. Solimena, M. & De Camilli, P. (1991) Trends Neurosci 14. 452-7.
5. Verge, C. F., et al. (1996) Diabetes 45, 926-933.
6. Bingley, P. J., et a/. (1997) Diabetes 46, 1701-1710.
7. Martin, S., et al. (2001) N Engl J Med 345, 1036-40.
8. Jaume, J. C., et a/. (2002) J Immunol 169, 665-72.
9. Banga, J. P., et al. (2004) Clin Exp Immunol 135, 74-84.
10. Reijonen, H., et al. (2000) Diabetes 49, 1621-6.
11. Harbers, S. O., et a/. (2007) J Clin Invest 117, 1361-9.
12. Hall, T. R., et al. (2007) Immunology*.* Nov 14 Epub ahead of print.
13. Padoa, C. J., et al. (2003) Diabetes 52, 2689-95.
14. Raju, R., et al. (2005) J Immunol 175, 7755-62.
15. Routsias, J. G., et al. (2003) J Autoimmun 21, 17-26.
16. Geha, R. S. (1985) Ric Clin Lab 15, 1-8.
17. Dwyer, D. S., et al. (1983) Nature 301, 611-4.
18. Lefvert, A. K. & Fulpius, B. W. (1984) Scand J Immunol 19, 485-9.
19. Taniguchi, O., et al. (1984) J Rheumatol 11, 291-7.
20. Birdsall, H. H. & Rossen, R. D. (1983) Clin Exp Immunol 53, 497-504.
21. Pan, Z. J., et al. (1998) J Clin Invest 102, 215-22.
22. Stafford, H. A., et al. (1995) J Immunol 155, 2754-61.
23. Anderson, C. J., et al. (1998) Arthritis Rheum 41, 33-40.
24. Chen, Q. Y., et al. (1999) Hepatology 29, 624-31.
25. Casiglia, D., et al. (1991) Diabetes Res 16, 181-4.
26. Elias, D., et al. (1990) Diabetes 39, 1467-71.
27. Elias, D., et al. (1984) J Biol Chem 259, 6416-9.
28. Jerne, N. K. (1974) Ann Immunol (Paris) 125C, 373-89.
29. Kassem, S. A., et al. (2000) Diabetes 49, 1325-33.
30. Finegood, D. T., et al. (1995) Diabetes 44, 249-56.
31. Turley, S., et al. (2003) J Exp Med 198, 1527-37.
32. Merbl, Y., et al. (2007) J CHn Invest 117, 712-8.
33. Shoenfeld, Y. (2004) Nat Med 10, 17-8.
34. Schlosser, M., et al. (2005) Diabetologia 48, 922-30.
35. Gilliam, L. K., et al. (2004) Clin Exp Immunol 138, 337-41.
36. Fenalti, G., et al. (2008) Diabetes. Jan 9 Epub ahead of print.
37. Kuhn, R. (2007) Pharmacotherapy 27,1412-24.
38. Kazatchkine, M. D. & Kaveri, S. V. (2001) N Engl J Med 345, 747-55.
39. Sapir, T. & Shoenfeld, Y. (2005) Clin Rev Allergy Immunol 29, 185-99.
40. Escher, R., et al. (2002) J Autoimmun 18, 71-81.
41. Borg, H. , et al. (2002) Diabetes 51 , 1754-62.
42. Decochez, K., et al. (2000) Diabetes Care 23, 838-44.
43. Savola, K., et al. (1998) Diabetologia 41, 1293-7.
44. Nayak, R., et al. (2001) Immunology 102, 387-95.
45. Brown, T. J. & Christie, M. R. (1993) Diabetologia Suppl 1, A92 (Abstr).
46. Tremble, J., et al. (1997) J CHn Endocrinol Metab 82, 2664-70.
47. Larrick, J. W. (1986) US Patent 4, 624,921.
48. Hampe, C. S., et al. (2000) J CHn Endocrinol Metab 85, 4671-4679.
49. Harlow, E. & Lane, D. (1988) Antibodies: A Laboratory Manual (Cold Spring Harbor Laboratory Press, Cold Spring Harbor, NY).

### Example 3: First degree relatives that later progress to T1D have less b96.11 - specific anti-Id

This example shows the analysis of GAD65Ab-negative sera from six individuals that later progressed to T1 D and 33 sera from GAD65Ab-negative individuals that remained non-diabetic. These samples were collected from first degree relatives of T1 D patients in the Children's Hospital of Pittsburgh Registry. The progressors had significantly lower levels of b96.11-specific anti-Id as compared to the non-progressors (p=0.04). These results show that individuals that progress to T1 D may have an early lack of anti-Id suggesting a predictive value of a subject's b96.11-specific anti-Id levels.

## Claims

1. A pharmaceutical composition comprising an anti-idiotypic antibody that specifically recognizes and binds an epitope of antibody b96.11 and, optionally, a pharmaceutically acceptable carrier, wherein the anti-idiotypic antibody competitively inhibits binding of b96.11 to GAD65 , wherein said antibody b96.11 has a light chain comprising the amino acid sequence shown in SEQ ID NO: 1 and a heavy chain comprising the amino acid sequence shown in SEQ ID NO: 3.

2. A pharmaceutical composition comprising a nucleic acid molecule that encodes an anti-idiotypic antibody of claim 1 and, optionally, a pharmaceutically acceptable carrier.

3. The composition of claim 1 or 2 for use in delaying the onset of diabetes in a subject.

4. The composition of claim 1 or 2 for use in inhibiting insulitis in a subject.

5. The composition for the use of claim 3 or 4, wherein the subject is a first degree relative of a Type 1 diabetes patient.

## Patentansprüche

1. Pharmazeutische Zusammensetzung, umfassend einen anti-idiotypischen Antikörper, der ein Epitop von Antikörper b96.11 spezifisch erkennt und bindet, und, optional, einen pharmazeutisch akzeptablen Träger, wobei der anti-idiotypische Antikörper die Bindung von b96.11 an GAD65 kompetitiv hemmt, wobei dieser Antikörper b96.11 eine leichte Kette, umfassend die in SEQ ID NO. 1 gezeigte Aminosäuresequenz, und eine schwere Kette, umfassend die in SEQ ID NO. 3 gezeigte Aminosäuresequenz, aufweist.

2. Pharmazeutische Zusammensetzung, umfassend ein Nukleinsäure-Molekül, das für einen anti-idiotypischen Antikörper nach Anspruch 1 kodiert, und optional, einen pharmazeutisch akzeptablen Träger.

3. Zusammensetzung gemäß Anspruch 1 oder 2 zur Verwendung im Verzögern des Ausbruchs von Diabetes bei einem Individuum.

4. Zusammensetzung gemäß Anspruch 1 oder 2 zur Verwendung im Hemmen von Insulitis bei einem Individuum.

5. Zusammensetzung zur Verwendung gemäß Anspruch 3 oder 4, wobei das Individuum ein Verwandter ersten Grades eines Patienten mit Typ 1-Diabetes ist.

## Revendications

1. Composition pharmaceutique comprenant un anticorps anti-idiotypique qui reconnaît et se lie spécifiquement à un épitope de l'anticorps b96.11 et, facultativement, un support pharmaceutiquement acceptable, l'anticorps anti-idiotypique inhibant de manière compétitive la liaison de b96.11 à GAD65, ledit anticorps b96.11 ayant une chaîne légère comprenant la séquence d'acides aminés représentée dans SEQ ID NO : 1 et une chaîne lourde comprenant la séquence d'acides aminés représentée dans SEQ ID NO : 3.

2. Composition pharmaceutique comprenant une molécule d'acide nucléique qui code pour un anticorps anti-idiotypique selon la revendication 1 et, facultativement, un support pharmaceutiquement acceptable.

3. Composition selon l'une des revendications 1 ou 2, pour une utilisation dans le retardement de l'apparition du diabète chez un sujet.

4. Composition selon l'une des revendications 1 ou 2, pour une utilisation dans l'inhibition de l'insulite chez un sujet.

5. Composition pour l'utilisation selon l'une des revendication 3 ou 4, dans laquelle le sujet est un parent de premier degré d'un patient atteint de diabète de Type 1.


### SEQUENCE LISTING

<110> University of Washington Hampe, Christiane S. Lernmark, Ake Oak, Shilpa
<120> MOLECULES AND METHODS FOR TREATMENT AND DETECTION OF DIABETES
<130> UW.22-WO-U1
<140> PCT/US08/________,________
   <141> 2008-09-06
<150> 60/970,409
   <151> 2007-09-06
<160> 4
<170> Patent In version 3.4
<210> 1
   <211> 217
   <212> PRT
   <213> homo sapiens
<400> 1
   Gln Ser Ala Leu Thr Gln Pro Ala Ser Ala Ser Gly Ser Pro Gly Gln
   1               5                   10                  15
   Ser Val Thr Ile Thr Cys Thr Gly Ser Ser Ser Asp Val Gly Gly Tyr
               20                  25                  30
   Lys Tyr Val Ser Trp Tyr Gln His His Pro Gly Lys Ala Pro Lys Leu
           35                  40                  45
   Met Ile Tyr Glu Val Ser Lys Arg Pro Ser Gly Val Pro Asp Arg Phe
       50                  55                  60
   Ser Gly Ser Lys Ser Gly Asn Met Ala Ser Leu Thr Val Ser Gly Leu
   65                  70                  75                  80
   Gln Ala Glu Asp Glu Ala Asp Tyr Tyr Cys Ser Ser Tyr Ala Gly Ser
                   85                  90                  95
   Tyr Asn Phe Tyr Val Phe Gly Asn Gly Thr Lys Val Thr Val Leu Gly
               100                 105                 110
   Gln Pro Lys Ala Asn Pro Thr Val Thr Leu Phe Pro Pro Ser Ser Glu
           115                 120                 125
   Glu Leu Gln Ala Asn Lys Ala Thr Leu Val Cys Leu Ile Ser Asp Phe
       130                 135                 140
   Tyr Pro Gly Ala Val Thr Val Ala Trp Lys Ala Asp Gly Ser Pro Val
   145                 150                 155                 160
   Lys Ala Gly Val Glu Thr Thr Lys Pro Ser Lys Gln Ser Asn Asn Lys
                   165                 170                 175
   Tyr Ala Ala Ser Ser Tyr Leu Ser Leu Thr Pro Glu Gln Trp Lys Ser
               180                 185                 190
   His Arg Ser Tyr Ser Cys Gln Val Thr His Glu Gly Ser Thr Val Glu
           195                 200                 205
   Lys Thr Val Ala Pro Thr Glu Cys Ser
       210                 215
<210> 2
   <211> 216
   <212> PRT
   <213> homo sapiens
<400> 2
   Gln Ser Val Leu Thr Gln Pro Pro Ser Ala Ser Gly Ile Pro Gly Gln
   1               5                   10                  15
   Arg Val Thr Ile Ser Cys Ser Gly Ser Arg Ser Asn Ile Arg Ser Gly
               20                  25                  30
   Phe Val Ser Trp Tyr Gln Gln Val Pro Gly Thr Ala Pro Lys Val Leu
           35                  40                  45
   Ile Asn Arg Asn Asp Gln Arg Pro Ser Gly Val Pro Asp Arg Phe Ser
       50                  55                  60
   Gly Ser Lys Ser Gly Thr Ser Ala Thr Leu Ala Ile Ser Gly Leu Arg
   65                  70                  75                  80
   Ser Glu Asp Glu Ala Asp Tyr Tyr Cys Ala Ala Trp Asp Asp Ser Leu
                   85                  90                  95
   Ser His Trp Val Phe Gly Gly Gly Thr Lys Leu Thr Val Leu Gly Gln
               100                 105                 110
   Pro Lys Ala Ala Pro Ser Val Thr Leu Phe Pro Pro Ser Ser Glu Glu
           115                 120                 125
   Leu Gln Ala Asn Lys Ala Thr Leu Val Cys Leu Ile Ser Asp Phe Tyr
       130                 135                 140
   Pro Gly Ala Val Thr Val Ala Trp Lys Ala Asp Ser Ser Pro Val Lys
   145                 150                 155                 160
   Ala Gly Val Glu Thr Thr Thr Pro Ser Lys Gln Ser Asn Asn Lys Tyr
                   165                 170                 175
   Ala Ala Ser Ser Tyr Leu Ser Leu Thr Pro Glu Gln Trp Lys Ser His
               180                 185                 190
   Lys Ser Tyr Ser Cys Gln Val Thr His Glu Gly Ser Thr Val Glu Lys
           195                 200                 205
   Thr Val Ala Pro Thr Glu Cys Ser
       210                 215
<210> 3
   <211> 228
   <212> PRT
   <213> homo sapiens
<400> 3
   Glu Val Gln Leu Val Glu Ser Gly Gly Gly Leu Val Gln Pro Gly Arg
   1               5                   10                  15
   Ser Leu Arg Leu Ser Cys Ser Ala Ser Gly Phe Thr Phe Gly Asp Tyr
               20                  25                  30
   Ala Met Ser Trp Phe Arg Leu Ala Pro Gly Lys Gly Leu Glu Trp Val
           35                  40                  45
   Gly Leu Ile Lys Ser Arg Ala Ile Asp Gly Thr Pro Gln Tyr Ala Ala
       50                  55                  60
   Ser Val Lys Gly Arg Phe Thr Ile Ser Arg Asp Asp Ser Asn Ser Ile
   65                  70                  75                  80
   Ala Tyr Leu Gln Met Asn Ser Leu Thr Thr Glu Asp Thr Ala Ile Tyr
                   85                  90                  95
   Tyr Cys Ala Arg Asp Phe Tyr Asp Phe Trp Asn Glu Phe Ser His Arg
               100                 105                 110
   Thr Phe Asp Phe Trp Gly Gln Gly Thr Leu Val Thr Val Ser Ser Ala
   115 120 125
   Ser Thr Lys Gly Pro Ser Val Phe Pro Leu Ala Pro Ser Ser Lys Ser
   130 135 140
   Thr Ser Gly Gly Thr Ala Ala Leu Gly Cys Leu Val Lys Asp Tyr Phe
   145 150 155 160
   Pro Glu Pro Val Thr Val Ser Trp Asn Ser Gly Ala Leu Thr Ser Gly
   165 170 175
   Val His Thr Phe Pro Ala Val Leu Gln Ser Ser Gly Leu Tyr Ser Leu
   180 185 190
   Ser Ser Val Val Thr Val Pro Ser Ser Ser Leu Gly Thr Gln Thr Tyr
   195 200 205
   Ile Cys Asn Val Asn His Lys Pro Ser Asn Thr Lys Val Asp Lys Arg
   210 215 220
   Val Glu Pro Lys
   225
<210> 4
   <211> 221
   <212> PRT
   <213> homo sapiens
<400> 4
   Gln Val Gln Leu Val Glu Tyr Gly Gly Gly Val Val Gln Pro Glu Arg
   1               5                   10                  15
   Ser Leu Arg Leu Ser Cys Gly Ala Ser Gly Phe Thr Phe Arg Ser Tyr
               20                  25                  30
   Gly Met His Trp Val Arg Gln Pro Pro Gly Lys Gly Leu Glu Trp Val
           35                  40                  45
   Ala Ser Ile Trp Tyr Asp Gly Asn Asn Lys Tyr Tyr Ala Glu Ser Val
       50                  55                  60
   Lys Gly Arg Phe Thr Ile Ser Arg Asp Asn Ser Asn Asn Thr Leu Tyr
   65                  70                  75                  80
   Leu Gln Met Asn Arg Leu Arg Thr Glu Asp Thr Ala Val Tyr Phe Cys
                   85                  90                  95
   Ala Arg Asp Glu Val Gly Gly Thr Leu Gly Ala Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys
       210                 215                 220